# EUROPEAN PATENT APPLICATION

(11) **EP 0 786 664 A1**
(43) Date of publication of application: **30.07.1997**
(21) Application number: 96400197.8
(22) Date of filing: 26.01.1996
(51) Int. Cl.: G01N 33/569, G01N 33/50

(54) **Diagnosis of opportunistic infections in aids patients**

(71) Applicant: IMMUNOTECH, F-13276 Marseille (FR)
(72) Inventor: Romagne, François, 42, Rue Henri-Tomasi, F-13009 Marseille (FR); Pellegrin, Jean-Luc, F-33700 Merignac (FR); Carron, Jean-Claude, F-33290 Parempuyre (FR); Moreau, Jean-François, F-33600 Pessac (FR)
(74) Representative: Simonnot, Bernard

(57) **Abstract**

Methods and compositions are disclosed herein for diagnosing opportunistic diseases, such as MAC (*Mycobacterium avium complex*) infection, toxoplasmosis and similar infections, in immunocompromised individuals. The disclosed methods and compositions generally employ optically detectable agents, such as fluorescence-conjugated antibodies or fragments thereof, and two- or three- color flow cytometry to characterize specific T-cell subpopulations present in body fluid (e.g., blood) and considered herein to be predictive of opportunistic disease. Specific embodiments involve assessing CD4-CD8- double negative (DN) T-cells or γδ+ T-cells free of the Vδ2 or the Vγ9 epitope. Fluctuations of such T-lymphocyte populations advantageously provide early indication of the presence of opportunistic intracellular pathogens, even in individuals such as AIDS patients who are refractory to diagnosis by conventional means.

## Description

This invention relates generally to methods and compositions for diagnosing opportunistic diseases in immunocompromised individuals. For example, the present invention provides methods and kits for diagnosing infection with Mycobacterium avium complex or Toxoplasma gondii.

### Background of the Invention

In the clinical management of patients with HIV infections, monoclonal antibodies and flow cytometry have significant clinical value. The receptor for HIV is the immunologically detectable CD4 molecule (Dalgleish et al. (1984) 312 Nature 763-766), mainly expressed by T-cells whose virus-mediated depletion over time induces an acquired immunodeficient state (Pantaleo et al. (1993) 328 N.E.J. Med. 327-334). CD4+cell counts, as determined by flow cytometry using monoclonal antibodies as detectable binding agents, thus are monitored routinely to follow disease progression. Such counts, when interpreted in the context of other clinical parameters, can identify a predisposition to opportunistic infections, which often occur at the late stages of the disease (Fahey et al. (1983) 308 N.E.J. Med. 842-843; Polk et al. (1987) 316 N.E.J. Med.62-66; Taylor et al. (1989) 3 J. Acquir. Immune Defic. Syndr. 114-124). Because of CD4+cell count is the most widely accepted and routine clinical parameter monitored in HIV seropositive patients, the status of other T-lymphocyte subpopulations heretofore generally has been ignored.

Recent studies suggest that T-cell subsets might be altered during HIV infection. In particular, expansions of T-cells expressing the γδ heterodimer T-cell receptor (γδ TCR) have been reported in HIV patients (Autran et al. (1989) 75 *Clin. Exp. Immunol.* 206-210; DePaoli et al. (1991) 83 *Clin. Exp. Immunol*. 187-191; Margolick et al. (1991) 58 *Clin. Immunopathol.* 126-138; DeMaria et al. (1992) 1665 *J. Infect. Dis.* 917-919; Hinz et al. (1994) 24 *Eur. J. Immunol*. 3044-3049; Boullier et al. (1995) 154 *J. Immunol*. 1418-1430). The possible physiological functions of γδ T-cells, which are classically CD4-/CD8- double negative (DN), remain largely unknown (Haas et al. (1993) 11 *Ann. Rev. Immunol*. 637-685). These cells have been observed to proliferate *in vitro* when exposed to certain tuberculous mycobacterial antigens and certain parasite extracts, and frequently are expanded *in vivo* in patients infected with tuberculous mycobacteria, plasmodium, toxoplasma or leishmania (Haas et al. (1993) 11 *Ann. Rev. Immunol*. 637-685; Ruiz et al. (1995) 22 *Cytometry* 211-216). Furthermore, reactivity of γδ T-cell subsets against highly conserved antigens, e.g., antigens expressed at specific stages of cell differentiation, might be consistent with a possible role in antitumor immunity (Haas et al. (1993) 11 *Ann. Rev. Immunol*. 637-685).

Many AIDS-defining diseases such as toxoplasmosis or Kaposi's sarcoma generally are amenable to diagnosis in clinical practice. However, this is not the case for *Mycobacterium avium complex* (MAC) infections. An infraclinical stage of the disease exists, which is refractory to diagnosis by the most widely used conventional method of mycobacterial blood cultures (Havlik et al. (1992) 165 *J*. *Infect. Dis*. 577-580). This has prompted the search for other clinical parameters allowing an early and reliable diagnosis of such infection in HIV seropositive patients (Chin et al. (1994) 19 *Clin. Infect. Dis*. 668-674). It is now well known, for example, that a CD4+ T-cell count below 50 per mm³ can be used to predict a mycobacteremia caused by MAC. Such a cell count, however, also indicates a severe immunodeficiency typically associated with the terminal stages of disease. Other clinical parameters such as a long term fever, lowered hematocrit, or a decreased concentration of serum albumin, can be used to predict MAC infection but these parameters are not pathogen-specific. Rather, such alterations in HIV infected patients can be multicausal. The identification of other immune parameters associated with, and/or predictive of specific opportunistic infections such as MAC advantageously would permit more timely implementation of both prophylactic and therapeutic strategies for disease management. Furthermore, because earlier detection of MAC colonization would allow a faster implementation of therapeutic intervention, survival of AIDS patients likely would be enhanced.

Needs remain for methodologies to specifically and rapidly diagnose opportunistic diseases which afflict immunocompromised individuals. Particular needs remain for methodologies that are useful for monitoring not only the onset of such diseases, but also disease progression. Additionally, particular needs remain for methodologies that are useful for monitoring opportunistic disease regression and the effectiveness of particular therapeutic regimens, as well as for monitoring disease relapse. Such methodologies would permit prompt clinical intervention and/or implementation of alternative therapeutic regimens.

It is an object of the instant invention to provide methods for rapid and reliable diagnosis of opportunistic diseases in immunocompromised individuals. It is another object to provide kits for rapid and reliable diagnosis of opportunistic pathogens such as nontuberculous mycobacterium and sporozoan parasites. It is yet another object of the instant invention to provide diagnostic methods and kits which can predict the onset of, progression of, and/or relapse from opportunistic diseases caused by *Mycobacterium avium complex, Toxoplasmosa gondii* and the like. This is accomplished, as disclosed herein, by determining the emergence and/or size of certain subpopulations of T-lymphocytes in an immunocompromised individual, such as an HIV-seropositive human.

### Summary of the Invention

It has been discovered that onset of, or subsequent relapse from, certain opportunistic diseases in immunocompromised individuals can be predicted by monitoring the expansion and/or emergence of particular subsets of T-lymphocytes. Based on this discovery, the present invention provides methods and kits for predicting disease by correlating the size of certain T-lymphocyte subpopulations in a blood sample with a particular opportunistic disease. An especially predictive subpopulation of T-lymphocytes is the γδ TCR+ lymphocyte. As disclosed herein, emergence and/or expansion of this subpopulation correlates with disease caused at least by *Mycobacterium avium complex* (MAC), *Toxoplasma gondii (T. gondii)*, and the like in immunocompromised individuals such as HIV-seropositive humans. The invention advantageously provides a rapid and direct mode of predicting disease caused by MAC, *T. gondii*, and the like, thus providing a significant improvement over existing diagnostic technology. Conveniently, the methods and kits disclosed herein achieve rapid diagnosis by exploiting clinically-accepted flow cytometry protocols and apparatus already available in hospitals and clinical laboratories.

Because the immunocompromised individual is especially predisposed to opportunistic disease, rapid and reliable diagnosis is critical to his or her physical well-being and, ultimately, to survival. Thus the invention described herein has particular relevance for the clinical management of individuals with congenital immune deficiency disorders, chemically- or virally-suppressed immune systems, or immune system deficiencies or disorders of other or unknown etiology. The invention achieves rapid and reliable diagnosis of opportunistic infections using a simple flow cytometry quantitation of selected T-lymphocyte populations, such as those displaying the γδ TCR. In this manner, results can be obtained in as little as thirty minutes after procuring an appropriate tissue or body fluid sample, such as a peripheral blood sample. In contrast, current methods for diagnosis of MAC infection require up to approximately two months to culture and positively identify the responsible organism. In the case of *T. gondii*, current diagnostic methods such as brain scans or retinal examination require up to approximately 24 hours and are only presumptive. The predictive value of such will be greatly improved by the instant invention. Central nervous tissue biopsy which is the reference for central nervous system toxoplasmosis is not a first intention diagnostic method. Moreover, the current methods often are labor-intensive and invasive.

A further limitation of current diagnostic methods of particular significance for diagnosis of the immunocompromised individual is that, in contrast to the present invention, they often rely on detection of circulating pathogen-reactive antibodies in a body fluid sample. Since immunocompromised individuals typically are deficient in circulating antibodies, such diagnostic approaches are unsuitable. AIDS patients, particularly asymptomatic AIDS patients (who may be afflicted with an infraclinical opportunistic infection), thus are particularly likely to benefit from the instant invention. Such patients may fail to exhibit overt clinical symptoms or humoral immunity normally attributable to the presence of opportunistic pathogens. Such difficulties in clinical management are obviated by practice of the instant invention, which relies on cellular indicators of disease, i.e., the population size of certain lymphocytes.

Broadly, the present invention features a method for assessing opportunistic disease by determining the size of a particular population of T-lymphocytes in a tissue sample from an immunocompromised individual. If for example, the size of the γδ TCR+ T-cell population is expanded relative to a reference standard, skilled artisans now will appreciate that treatment is warranted to control infection by an intracellular pathogen. In two currently preferred embodiments, the invention is practiced to predict disease associated with exposure to nontuberculous mycobacteria such as MAC, or a sporozoan parasite such as *T. gondii*. Generally, the methods disclosed herein rely on optical detection to determine the size of T-lymphocyte populations, e.g., using fluorescent agents and two- or three-color flow cytometry apparatus.

Preferably, the detected T-lymphocytes display γδ T cell receptors lacking a Vδ2 polypeptide or a Vγ9 polypeptide. Expansion of such a lymphocyte population is predictive of exposure to an opportunistic pathogen, such that clinical treatment therefor is warranted. Alternatively, the method involves determining the ratio of lymphocytes displaying a Vδ2 polypeptide or a Vγ9 polypeptide to those lacking a Vδ2 polypeptide or a Vγ9 polypeptide, respectively. Thus, the present invention allows detection of an inversion of such population ratios, an event that also is predictive of infection by an opportunistic pathogen such as MAC. Optionally, the foregoing and other embodiments of the invention can be applied in lieu of or to corroborate other diagnostic techniques.

In yet another embodiment, the invention involves determining the size of a population of T-lymphocytes displaying either the CD3 antigen or the αβ T cell receptor, but lacking the CD4 and CD8 antigens. Emergence and/or expansion of this particular subset of T-lymphocytes similarly is predictive of disease caused by an opportunistic nontuberculous mycobacterium or a sporozoan parasite.

Still other embodiments of the invention involve combinations of the above-described methods. For example, one embodiment involves determining the size of a population of lymphocytes displaying a CD3+/CD4-/CD8-/γδ+ or a αβ+/CD4-/CD8- immunophenotype. Another embodiment involves determining the size of lymphocyte populations displaying a CD3+/CD4-/CD8-/γδ+/Vδ2- or CD3+/CD4-/CD8-/γδ+/Vγ9- immunophenotype. Emergence and/or expansion of lymphocyte populations displaying such phenotypes is considered herein to be predictive of opportunistic disease.

When practicing the instant invention, a tissue sample containing lymphocytes is contacted with one or more detectable agents that bind selectively to particular subsets of T-lymphocytes. After a suitable incubation period, T-lymphocyte population(s) bound by these agent(s) are detected and, preferably, counted. Detectable selective binding agents useful herein include, for example, agents that bind to T-lymphocytes displaying: γδ T cell receptors, including preferably γδ T cell receptors lacking a Vδ2 polypeptide or a Vγ9 polypeptide. Other detectable selective binding agents useful herein include agent(s) for identifying the CD3+/CD4-/CD8-, αβ+ CD4-/CD8-, CD3+/CD4-/CD8-/γδ+, CD3+/CD4-/CD8-/γδ+/Vδ2-, and/or CD3+/CD4-/CD8-/γδ+/Vγ9-immunophenotypes. In many embodiments, the selective binding agents are immunologically derived, such as antibodies (polyclonal or monoclonal) or antigen binding fragments thereof, including divalent or monovalent fragments such as Fv fragment. The present binding agents are directly or indirectly detectable by optical means such as flow cytometry, immunohistochemistry or the like. The present binding agents can be used alone or in combination, as desired. The present binding agents can be used to determine emergence or expansion of particular subsets of lymphocytes, or to determine an inversion of the ratio of particular lymphocyte subsets.

In one embodiment, the invention involves monitoring, at suitable time intervals, changes in the size of a particular subset of T-lymphocytes, e.g., γδ+ cells. This embodiment involves determining the size of a population of T-lymphocytes displaying one of the above-described immunophenotypes in at least two different tissue samples comprising lymphocytes obtained at two different times from the same immunocompromised individual. This embodiment permits the identification of an emergent opportunistic disease before significant clinical progression has occurred. This embodiment is particularly well-suited for predicting disease in an asymptomatic AIDS patient who otherwise may be refractory to diagnosis using conventional clinical parameters based on monitoring levels of hematocrit, serum albumin, and the like.

In another aspect, the invention provides kits for immunophenotyping lymphocytes according to the above-described methods. In certain embodiments, the kits of the instant invention contain an appropriate reference standard for monitoring changes in the size of one or more of the above-described populations of lymphocytes. Other embodiments permit assessment of ratio inversions among populations of T-lymphocytes.

Certain preferred kits include binding agents for detecting optically T-lymphocytes displaying a γδ T cell receptor, preferably lacking a Vδ2 polypeptide or a Vγ9 polypeptide. Other preferred kits include binding agents for detecting optically T-lymphocytes displaying at least one cell surface antigen selected from CD3 or CD8, preferably displaying a CD3+/CD4-/CD8- immunophenotype. Currently preferred kits including fluorescent selective binding agents suitable for two- or three-color flow cytometry protocols.

The foregoing and other objects, features and advantages of the present invention will be made more apparent from the following detailed description of preferred embodiments of the invention.

### Brief Description of the Drawings

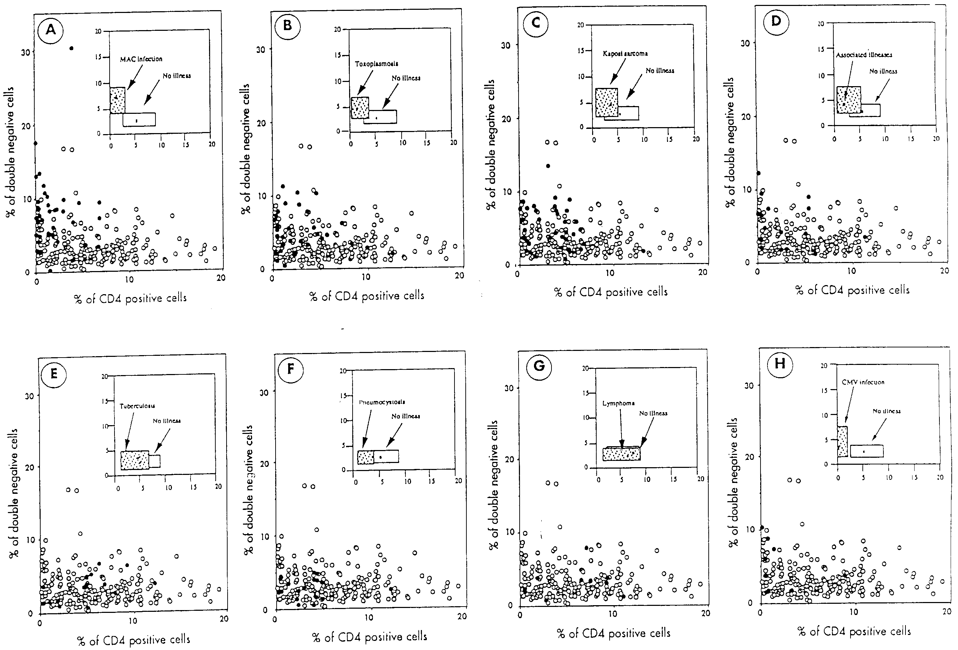

The foregoing and other objects, features and advantages of the present invention, as well as the invention itself, will be understood more frilly from the following description of preferred embodiments, when read together with the accompanying drawing, in which:
The Figure (Panels A-H) depicts a series of plots characterizing the various T-cell populations associated with a particular disease. The percentages of CD4+ are plotted on the X axis, against the percentages of DN T-cells on the Y axis, for patients expressing a given pathology (closed circles) vs. asymptomatic patients (n=216) (open circles). The inserts illustrate the median value for each group and the area covered by the 25th and the 75th percentiles as well as the median for the same parameters: Panel A - *Mycobacterium avium complex* infections (n=40); Panel B - *Toxoplasmosis gondii* (n=34); Panel C - Kaposi's sarcoma (n=39); Panel D - Associated illnesses (n=17); Panel E - Tuberculosis (n=15); Panel F - Pneumocystosis (n=19); Panel G - Lymphomas (n=10); Panel H - Cytomegalovirus infections (n=9).

### Detailed Description of the Invention

The invention described herein capitalizes on the discovery that the size of, or emergence of, certain populations of T-lymphocytes in immunocompromised individuals predicts onset of certain opportunistic diseases. That is, expansion of certain lymphocyte populations or ratio inversions among certain subsets of T-lymphocytes indicates onset, progression, and/or relapse from certain opportunistic infections which afflict immunocompromised individuals.

The methods and compositions of the present invention exploit this discovery to advantage in a variety of aspects and embodiments as set forth herein. Generally, methods and compositions of the present invention provide the skilled practitioner with the analytical tools and technical know-how sufficient to rapidly diagnose disease caused by certain opportunistic pathogens in immunocompromised individuals. Guidance provided herein accordingly will facilitate clinical assessments, thereby enhancing the beneficial effects of therapeutic treatments initiated to ameliorate and/or control such diseases, particularly opportunistic diseases caused by *Mycobacterium avium complex* (MAC) or *Toxoplasma gondii*, and similar pathogens.

Before proceeding further with a detailed description of the currently preferred embodiments of the instant invention, an explanation of certain terms and phrases will be provided. Accordingly, it is understood that each of the terms set forth below is defined herein at least as follows:

"Immune deficiency syndrome" means a disorder of the immune system in which an individual's physiological response to a particular antigen or immunogenic stimulant is abnormal or atypical relative to an art-recognized norm, such as, for example, an age-matched/gender-matched healthy individual. An individual afflicted with an immune deficiency disorder is typically hyporesponsive to a given antigen or immunogenic stimulant. As contemplated herein, an immune deficiency syndrome can be congenital in origin or acquired. An acquired immune deficiency can result from contact with and/or exposure to chemical and/or biological immunosuppresive agents, such as chemotherapeutic agents, e.g. cyclosporin, and microbiotic agents, e.g. HIV-1, and the like. Moreover, an immune deficiency as contemplated herein can be reversible or irreversible.

"Immunocompromised" mammal, in contrast to "immunocompetent" mammal, is one unable to mount a normal physiological response to an antigen or immunogenic stimulant. That is, such a mammal's competence to respond to immunologic challenge has been compromised, e.g., by an immune deficiency syndrome. As disclosed herein, practice of the instant invention has particular utility for diagnosis of immunocompromised mammals because of their predisposition to infection by certain opportunistic pathogens, coupled will, their unsuitability for traditional diagnostic methodologies. As contemplated herein, an immunocompromised mammal can be afflicted with a congenital immune deficiency syndrome. Alternatively, for example, an immunocompromised mammal can be undergoing treatment with an immunosuppresive drug such as, for example, drug therapy as an adjunct to organ transplantation. Further, an immunocompromised mammal can be afflicted with a biological agent which induces an immunosuppressed state, such as for example the HIV-1 virus. Preferably, the invention may be used for diagnosis of an immunocompromised primate; more preferably it may be used for diagnosis of an immunocompromised human.

"Opportunistic intracellular pathogen" means any organism able to cause opportunistic disease by invading and colonizing living cells in a mammal, particularly an immunocompromised mammal. That is, an opportunistic intracellular pathogen is one that invades an immunocompromised mammal, and subsequently causes a localized or, preferably, a disseminated opportunistic infection because the immunocompromised individual fails to mount an immune response sufficient to contain and/or curtail pathogen colonization. As contemplated herein, one type of opportunistic intracellular pathogen particularly susceptible to diagnosis using the instant invention is a nontuberculous mycobacterium. Another type of opportunistic intracellular pathogen particularly susceptible to diagnosis as disclosed herein is a sporozoan parasite. Both of these opportunistic intracellular pathogens can cause "opportunistic disease" in an immunocompromised mammal which can be predicted through practice of the instant invention.

"Nontuberculous mycobacterium" includes any mycobacterium which is not the causative agent of tuberculosis and is not a member of the tuberculosis complex, i.e., is not *Mycobacterium tuberculosis, M.bovis, M.africanum* or *M.microti.* As is well-known in the art, the acid-staining characteristics alone of tuberculous mycobacteria can not be used to distinguish them from the nontuberculous mycobacteria. Rather, a definitive identification involves assessment of a combination of cultural, biochemical and antigenic features. As described in Stein, ed. (1994) *Internal Medicine* (4th ed., Moslay-Year Book, Inc., St. Louis, MO), the disclosure of which is herein incorporated by reference, one identification method is the Runyon classification system which utilizes light-induced pigmentation to identify the most clinically prevalent nontuberculous mycobacterium. For example, *Mycobacterium avium complex* (MAC) is categorized as a non-chromogen in group III. *Mycobacterium avium complex* contains a large number of serotypically distinct strains that are otherwise difficult to separate. Clinically speaking, nontuberculous mycobacteria typically are of low virulence, infrequently causing disseminated disease in immunocompetent individuals. For example, in immunocompetent individuals, nontuberculous mycobacterial diseases remain localized and progress slowly, and constitutional symptoms are not prominent. In contrast, more classical manifestations of opportunistic disease caused by nontuberculous mycobacterium occur in immunocompromised individuals, including AIDS patients. In such patients, clinical manifestations of opportunistic disease caused by nontuberculous mycobacteria include: disseminated pulmonary disease and lymphatic disease, including involvement of blood, bone marrow and a variety of viscera, as well as very high numbers of bacilli with little granulomatous response. Up to 24% of AIDS patients have opportunistic disease caused by *M. avium complex*; the mortality rate for these individuals is high. Diagnosis conventionally is made by culturing the organism, usually from the blood. The time interval for a definitive culture result is approximately two weeks, at least, and can be up to eight weeks. Generally, other traditional techniques for diagnosing mycobacteria, such as myeloculture and/or tissue biopsy, can also take up to three weeks and can require up to an additional six weeks for confirmation of a negative test. As disclosed herein, the instant invention is particularly suitable for rapid diagnosis of disease caused by nontuberculous mycobacterium including *Mycobacterium avium complex, M. avium intracellulare, M. kansasii, M. scrofulaceum, M. ulcerans, M. marinum, M. xenopi, M. szulgai, M. simiae, M. hemophilium, M. genovensee, M. fortuitum, or M. chelonei.* As earlier stated, diagnosis of infection with the foregoing pathogens can be achieved in approximately 30 minutes through the practice of the instant invention.

"Sporozoan parasite" includes any obligate intracellular parasite of the Phylum *Apicomplexa*, Class *Sporozoea,* particularly any one or more of the Genus *Leishmania, Isospora, Toxoplasma, Plasmodium, Babesia, Cryptosporidium* or *Sarcocystis*. A sporozoan parasite particularly suitable for diagnosis using the instant invention is *Toxoplasma gondii*. As taught by Stein (1994) (incorporated by reference above), *T. gondii* is ubiquitous in nature and is especially problematic for immunocompromised human hosts. In immunocompetent individuals, antibody and complement can kill extracellular trophozoites, but cell-mediated immunity is required to stop proliferation of intracellular organisms. Thus, if an immune system is disturbed by an illness such as AIDS or by immunosuppressive therapy, dormant tissue cysts which rupture can thereafter cause disseminated disease. Thus the pathologic manifestations vary with the host's immune status. In AIDS patients, central nervous system involvement is common; a mass lesion(s) in the brain, particularly in the gray matter of the cortex and the basal ganglia, is indicative of a toxoplasmic brain abscess. Central nervous system manifestations of toxoplasmosis occur in 10-25% of AIDS patients. Ocular toxoplasmosis also occurs. In addition, a toxoplasmosis infection can be acquired *in utero*, thus toxoplasmosis also can be congenital in origin. In conventional methods of diagnosis of toxoplasmosis, serologic tests for antibodies are generally coupled with histology evaluations. While this is effective for the immunocompetent individual, it is not adequate for the immunocompromised individual because antibody responses are depressed in the immunocompromised individual. Accordingly, art-recognized seroconversion assays or IgG assays are not reliable for diagnosis of immunocompromised individuals. The case of congenital *in utero* toxoplasmosis is further complicated by the fact that maternal antibodies reactive with *Toxoplasma* can persist in the infant up to 12 months after birth. With regard to ocular toxoplasmosis, diagnosis typically is based on observing retinal lesions. Rapid diagnosis of opportunistic disease by sporozoan parasites including *Leishmania, Isospora, Toxoplasma, Plasmodium, Babesia, Cryptosporidium* or *Sarcocystis* can be made through practice of the present invention.

"Detectable" agent means any agent which can be detected, directly or indirectly. Moreover, a detectable agent as contemplated herein is a binding agent, i.e., an agent which can form selectively a stable complex with a desired target substance. As disclosed herein, one target substance to which the detectable agent selectively binds is preferably an antigen expressed by lymphocytes, most preferably a cell-surface antigen such as, for example, the γδ TCR described below in more detail. To practice the instant invention, all that is required is that a detectable agent selectively bind to a desired target substance, thereby permitting quantitation of the target substance. The complex formed between the agent and target substance can be stabilized by covalent or non-covalent bonds. In a currently preferred embodiment, the agent is detectable optically. In a particularly preferred embodiment, an optically detectable agent is used together with conventional flow cytometry apparatus. In certain preferred embodiments, flow cytometry is conducted using detectable agents which are directly conjugated with fluorescent moieties and are detectable by at least 2- and/or 3-color flow cytometry. For example, as is well-known in the art, an antibody can be conjugated with a fluorescent moiety. In yet other embodiments, a detectable agent is indirectly detectable, i.e., the agent is rendered detectable by association with a second reagent conjugated to a detectable moiety. A preferred detectable agent is a specific-binding protein such as, but not limited to, an antibody, or a monovalent or divalent antigen-binding fragment thereof. Another specific-binding protein useful in practice of the instant invention comprises an antigen-specific immunoglobulin Fv region. An antibody, fragment, or Fv region can be either monoclonal or polyclonal in origin and can be rendered detectable by conjugation with a directly detectable moiety such as, but not limited to, an enzyme, a radionuclide, a fluorophore or a chromophore. Specific embodiments are described below in more detail. Alternatively, indirect detection of a specific-binding protein such as an antibody also can be accomplished using multiple antibodies and/or non-immunoglobulin binding pairs such as, but not limited to, the biotin/avidin pair. Indirect detection methods are well-known in the art. For example, a first binding agent can be rendered detectable by further contacting it with an immunoconjugate selected from a species-specific antibody, or an Fc binding protein such as the well-known protein A or protein G. In this instance, the immunoconjugate would further comprise a detectable moiety such as an enzyme, a radionuclide, a fluorophore or a chromophore. Again, specific embodiments are described below in more detail. In still other embodiments, the agent is detectable because of its physical characteristics, e.g., size or light-scattering properties, alone or when complexed with a target substance. Equivalents of directly detectable or indirectly detectable agents useful in the instant invention will be identified easily by the artisan using only routine skill and experimentation.

"Immunophenotype" or "phenotype" as used herein is understood to mean the antigenic characterization of a lymphocyte such as a T-cell. That is, the characterization of a T-cell's surface antigens expressed in terms of its antigenic or immunogenic components. As will be appreciated by the skilled artisan, the variety of cells involved in the immune response and their cellular lineages have been extensively characterized using cell surface antigens as specific cellular identification markers and/or response-monitoring tools. For example, a major surface antigen typical of mature T helper cells is the CD4 antigen (see below). Immunologically speaking, a mature T helper cell can either lack the CD4 antigen, i.e., have a CD4- immunophenotype, or can display the CD4 antigen, i.e., have a CD4+ immunophenotype. Thus a cell's immunophenotype refers to a configuration of one or more cell surface antigens displayed by the cell. Cell surface antigens can be characterized or described using immunological reagents such as antibodies, or using any other sufficiently selective binding agent such as a receptor-ligand pair, lectin-oligosaccharide pair, or the like. Thus, as described below in greater detail, the instant invention is suitable for determining a variety of lymphocyte immunophenotypes as predictors of opportunistic diseases such as MAC, toxoplasmosis and the like.

"CD3," "CD4," "CD8," "CD14," and "CD45" mean the art-recognized cluster designations 3, 4, 8, 14, and 45 respectively. "Cluster designations" or "CDs" are the abbreviations currently used to define certain surface antigens characteristic of T-cells. The art-recognized designations for human leucocyte differentiation antigens designations used throughout this disclosure were derived by the Leucocyte Workshop Study Sections of the 5th International Conference, Boston, Massachusetts (November 1993). As disclosed herein, one currently preferred T-cell population contains T-cells displaying the CD3 antigen, i.e., these cells have a CD3+ immunophenotype which is characteristic of mature T-cells. The presence of CD3 antigen can be determined using suitable detectable agents such as those commercially available from Becton-Dickinson Co. (Mountainview, CA) and Immunotech (France). For example, one suitable detectable agent is an anti-CD3 monoclonal antibody conjugated with peridinin-chlorophyll (Per-CP). Other suitable exemplary agents are described herein below.

CD4 surface antigens are characteristic of mature T helper cells, in particular, those T-cells bearing the HIV antigen receptor. It is well-known that, subsequent to HIV-1 infections, CD4+ cells become depleted in certain HIV-seropositive individuals. As disclosed herein, one currently preferred tissue sample contains a predominance of T-helper cells lacking the CD4 antigen, i.e., these cells have a CD4- immunophenotype. Currently, a tissue sample containing <400 CD4+ cells/mm³, more preferably <200 CD4+ cells/mm³, is suitable for use with the instant invention. In certain embodiments, a tissue sample containing <100 CD4+ cells/mm³, preferably <50 CD4+ cells/mm³, is suitable. As disclosed herein, certain embodiments of the instant method are practiced by determining both the CD4-deficient, i.e., CD4-, and the CD4+ lymphocyte populations in a tissue sample. As exemplified below, the presence and/or absence of the CD4 antigen can be determined using suitable detectable agents such as those commercially available from Becton-Dickinson Co. (Mountainview, CA) and Immunotech (France). For example, one such agent is an anti-CD4 monoclonal antibody conjugated with fluorescein isothiocyante (FITC).

CD8 surface antigen is characteristic of T cytotoxic/suppressor cells which assume a prominent role in cell-mediated immune responsiveness in immunocompetent individuals. As disclosed herein, one currently preferred T-cell population is lacking surface expression of the CD8 antigen, i.e., these cells have a CD8- immunophenotype. As exemplified below, a CD8-deficient T-cell population can be identified using agents such as those commercially available from Becton-Dickinson Co. (Mountainview, CA) and Immunotech (France). For example, one suitable agent is an anti-CD8 monoclonal antibody conjugated with fluorescein isothiocyante (FITC). Other suitable exemplary agents are described herein below.

CD14 is the surface antigen characteristic of monocytes. As disclosed herein, cells displaying the CD14 antigen, i.e., CD14+ cells, are excluded from the tissue sample population which is ultimately used to determine the immunophenotypes described herein as preferred. That is, a general population lacking the CD14, i.e., having a CD14- immunophentoype, is ultimately used. The CD14 antigen is a control marker useful for establishing acceptable flow cytometry parameters for gating lymphocytes. The proper use of this control, or its equivalent, for this purpose will be appreciated by one of skill in the art. One suitable agent for determining CD14- cells is an anti-CD14 monoclonal antibody (Becton-Dickinson Co., Mountainview CA) conjugated with phycoerythrin (PE).

CD45 is a surface antigen characteristic of leukocytes, i.e., pan leukocytes, including at least the well-known antigens T2000 and LCA. As disclosed herein, cells displaying the CD45 antigen, i.e., CD45+ cells, are included in the tissue sample population which is ultimately used to determine the T-cell immunophenotypes described herein as preferred. The CD45 antigen is another control marker useful for establishing acceptable flow cytometry parameters for gating lymphocytes. The proper use of this control, or its equivalent, for this purpose will be appreciated by one of skill in the art. One suitable agent for determining CD45+ cells is an anti-CD45 monoclonal antibody (Becton-Dickinson Co., Mountainview CA) conjugated with FITC. Skilled artisans will recognize that there are many suitable equivalents of the aforementioned agents and detectable moieties.

"αβ T cell receptor" means the art-recognized designation currently used to define a clone-specific disulfide-linked heterodimer on the T-cell surface composed of the T-cell receptor (TCR) subunits α and β. Currently, two different TCRs are known; one is the αβ TCR, the other is the "γδ T-cell receptor" (γδ TCR). In some embodiments, the instant invention is practiced using a population of T-cells having an αβ- immunophenotype, i.e., T-cells deficient in the αβ TCR. As disclosed herein, an αβ deficient T-cell population can be identified using suitable agents such as those commercially available from Becton-Dickinson Co. (Mountainview, CA). For example, one suitable agent is an anti-TCR αβ monoclonal antibody conjugated with phycoerythrin (PE). Other suitable exemplary agents are described herein below.

As used herein, the γδ TCR means a clone-specific disulfide-linked heterodimer on the T-cell surface composed of T-cell receptor (TCR) subunits γ and δ. The term "pan γδ" or "panδ" includes all T-cells having the γδ T-cell receptor on their surface. Certain embodiments of the instant invention are particularly suitable for determining the size of a population of T-cells displaying at least a γδ TCR, i.e., T-cells having a γδ+ immunophenotype. As will be appreciated by the skilled artisan, cells expressing the γδ+ immunophenotype are typically CD4- CD8- double negative (DN) T-cells. Additionally, as will also be appreciated by the skilled artisan, γδ+ T-cells are typically of the αβ-immunophenotype. Certain other embodiments of the instant invention are particularly suitable for determining the relative sizes of different T-cell populations having at least a γδ+ immunophenotype and at least a γδ-immunophentoype.

In some embodiments, immunophenotypes representative of other γδ polypeptide components, i.e., epitopes of the γδ TCR, are preferred. For example, T-cells bearing the γδ TCR but lacking the "Vγ9 polypeptide" (i.e., Vγ9-) are preferred. In still other embodiments, other different epitopes of the γδ TCR are preferred. For example, T-cells bearing the γδ TCR but lacking the "Vδ2 polypeptide" (i.e., Vδ2-) can be preferred. In yet other embodiments, practice of the instant invention features determining the relative size of at least two different T-cell populations, one lacking such eptitopes and one having such epitopes, i.e., determining the relative distribution of Vγ9- to Vγ9+ cells and/or Vδ2- to Vδ2+ cells. Such determinations are exemplified and described in detail below. As disclosed herein, a γδ+ immunophenotype as well as a γδ- immunophenotype within a T-cell population can be identified using suitable agents such as those commercially available from Immunotech (France). For example, one suitable agent is an anti-TCR γδ monoclonal antibody conjugated with phycoerythrin (PE). Other suitable agents include anti-Vδ2 and anti-Vγ9 monoclonal antibodies available from Immunotech. Skilled artisans will appreciate that there are many suitable equivalents of the aforementioned agents and detectable moieties. Preparation of other similar agents suitable for determining γδ or αβ TCRs (and/or epitopes thereof) is possible using only routine skill and experimentation together with knowledge available to the art.

"Tissue sample" includes any body tissue sample comprising lymphoid cells, preferably T-cell lymphocytes characteristic of lymphocytes associated with cellular immune responsiveness. Peripheral blood is a currently preferred tissue. Optionally, peripheral blood can be heparinized. A tissue sample can be obtained from an immunocompromised and/or immunocompetent mammal using routine procedures. As contemplated herein, T-cells in a preferred tissue sample display, or can be induced to display, at least one of the following surface antigens: CD45 (pan leukocyte, i.e., T2000; LCA); CD3 (thymocytes; mature T-cells; associated with T-cell receptor antigens); CD4 (T-helper/inducer; receptor for HIV antigen); CD8 (T-cytotoxic/suppressor cells). In some currently preferred embodiments, T-cells in a suitable tissue sample display, or at least are capable of displaying upon appropriate activation the above-described, clone-specific TCRs and their respective epitopes.

As disclosed herein, the instant invention provides methods and compositions for determining, in a suitable tissue sample, the size of T-cell populations displaying at least one, preferably at least two, of the above-described surface markers. When practicing the instant invention, the identity of the particular markers and/or immunophenotype(s) to be determined varies depending on the T-cell population of interest to the skilled artisan under the circumstances. Together with this disclosure, one of ordinary skill in the art would be able to determine the identity of the particular markers relevant to the circumstances at hand using only routine experimentation. Currently preferred markers are exemplified below.

"Reference standard" refers to any specimen, kit component, or exemplar which can provide the skilled artisan with a normative value useful for identifying an expanded T-cell population in a tissue sample. A particularly suitable reference standard is an historical quantitative and/or qualitative value obtained from a retrospective study of normal and/or immunocompromised individuals without infection versus immunocompromised individuals with infection. For example, the reference standard can be the art-recognized norm described above. As contemplated herein, the reference standard can be naturally-occurring or fabricated. For example, a tissue sample from an immunocompetent mammal can provide a standard against which a test sample from an immunocompromised mammal is compared. Any significant alteration in the size and/or distribution of the preferred immunophenotypes would be indicative of disease. Alternatively, a tissue sample from an immunocompromised mammal determined by routine clinical procedures to be free of opportunistic infection would provide a suitable reference standard against which a test sample can be compared. Again, any significant alteration in the size and/or distribution of T-cell populations displaying the preferred immunophenotypes would be indicative of disease as disclosed herein. Yet another suitable reference standard for those specific embodiments which practice monitoring an immunocompromised mammal over time is the earliest-drawn tissue sample from that individual. In essence, the first sample within a series of samples drawn over time provides the skilled artisan with a base-line value against which alterations or perturbations in established T-cell populations can be detected over time. All of these specimens provide the skilled artisan with a reference material suitable for use with any of the disclosed embodiments of the instant invention.

"Means for determining the size of a population of lymphocytes" includes any means suitable for use with the disclosed embodiments of the instant invention. For example, a laser-equipped flow cytometer can be used to quantitate T-cell populations previously contacted with, and selectively bound by, fluorescent binding agents as described herein. However, all that is required to practice the instant invention is a means which provides the skilled artisan with at least an ability to determine the size of a preferred population of lymphocytes, such that when the means is applied to a tissue sample and/or a reference standard, an expansion of, or other perturbation in, the size of the population is detectable. For example, fluorescence microscopy and laser scanning imaging are suitable means by which the skilled artisan can practice the instant invention.

As earlier described, it has been discovered that onset of, progression of, and/or subsequent relapse from certain opportunistic diseases in immunocompromised individuals can be predicted by monitoring the size and/or emergence of particular populations of T-lymphocytes. The present invention exploits this discovery to provide diagnostic methods and kits for assessing the disease state of an immunocompromised individual.

Accordingly, in a broad aspect, the instant invention features a diagnostic method for determining whether the size of a particular population of T-lymphocytes in a tissue sample from an immunocompromised individual is altered relative to a suitable reference standard. As disclosed herein, expansion and/or emergence of certain T-cell populations is indicative of disease caused by infection with certain intracellular pathogens. Such alterations in the size of certain T-cell populations also indicate that treatment of the immunocompromised individual is warranted to control progression of the infection. As further disclosed herein, a decline in certain other T-cell populations also is indicative of opportunistic diseases. Taken together, these alterations in the size of T-cell populations also permit the skilled artisan to monitor the ratios of certain T-cells, with certain ratio inversions also being indicative of opportunistic disease as disclosed herein.

Practice of the instant method involves at least: providing a tissue sample comprising lymphocytes; contacting the sample with at least one detectable agent that binds selectively to T-lymphocytes displaying a preferred immunophentoype such as, for example, a γδ T-cell receptor; and, detecting lymphocytes bound selectively by at least one said agent. As disclosed herein, two or more detectable agents that each bind selectively to different T-cells, and up to at least five agents in some preferred embodiments, can also be used to predict disease. This type of multi-parameter immunophenotyping permits the skilled artisan to successively characterize certain preferred subpopulations of T-cells, thereby constructing a hierarchy of immunophenotypes among related subpopulations. As contemplated herein, such a hierarchy can be used to successively confirm a diagnosis. For example, the present invention provides a method for detecting lymphocytes with a CD3+/CD4-/CD8- phenotype using a first, second and third agent which binds selectively to cells displaying the CD3, CD4 and CD8 antigens, respectively. The population of CD3+/CD4-/CD8- cells is then contacted with a fourth agent which binds selectively to cells also displaying the γδ+ immunophenotype. These CD3+/CD4-/CD8-/γδ+ cells are, in turn, contacted with a filth detectable agent which binds selectively to the Vδ2 epitope. In some embodiments, the filth agent binds instead the Vγ9 epitope. The resulting cumulative immunophenotype of CD3+/CD4-/CD8-/γδ+/Vδ2- (or CD3+/CD4-/CD8-/γδ+/Vγ9-) is also clinically significant because of its predictive value as disclosed herein below. Thus this type of successive immunophenotyping permits the skilled artisan to determine the size of multiple T-cell subpopulations, each of which alone is clinically significant as a predictor of opportunistic disease but which, when cumulatively considered, have additional clinical significance for their confirmatory value. It is contemplated that this type of successive immunophenotyping will be particularly useful for diagnosing disease in immunocompromised individuals in whom certain T-cell subpopulations appear unaltered relative to other immunocompromised individuals.

One currently preferred embodiment of the invention involves monitoring the size of a particular subset of T-lymphocytes over time. This is accomplished by repeating, at least once, the above-described steps using at least two different samples of lymphocytes obtained at two different times from the same immunocompromised individual. In certain instances, this embodiment is practiced until an alteration in a particular T-cell population, such as an expansion, is detected. This embodiment permits identifiying certain diseases as they emerge and before significant progression has occurred. This embodiment also permits the earlier samples to be used as internal reference standards relative to which assessments of population declines, expansions and/or inversions over time can be made by the clinical practitioner. Alternatively, external reference standards such as those described herein can be used, either alone or in combination with the immunocompromised individual's own tissue sample.

The present invention contemplates determining the size of a T-cell population using both subtractive and additive methods of detection. The present invention further contemplates both direct and indirect methods for determining the size of a T-cell population. For example, as described above, agents which bind selectively to the CD3, CD4 and CD8 antigens can be used to detect the CD3+/CD4-/CD8- immunophenotype. In this instance, the CD3+ phenotype is determined by directly measuring the bound CD3 agent. This is an affirmative or direct type of detection. In contrast, the CD4- or CD8- phenotype is determined by indirectly measuring populations of cells to which no agent is bound, i.e., cells which do not bind the CD4 or CD8 agent and are presumed to be CD4- and CD8-. Thus, using a subtractive analysis, CD4-, CD8- cells can be calculated by comparing the size of the total cell population to the size of the subpopulation selectively bound by the agents. The T-cell population displaying a CD3+/CD4-/CD8- phenotype is ultimately determined by combining these indirect and direct measurements using an additive analysis, for example. Numerous permutations of this scenario are possible. It will be appreciated by the skilled artisan that various combinations of additive and subtractive measurements can be used when practicing the instant invention. Relying on routine experimentation and the knowledge already in the art, the skilled artisan will recognize equivalent methods and binding agents suitable for detecting the preferred T-cell populations disclosed herein.

In certain embodiments, the instant method detects emerging and/or expanded populations of T-lymphocytes displaying γδ T cell receptors. In certain other embodiments, the detected T-lymphocytes display γδ T cell receptors lacking a Vδ2 polypeptide or a Vγ9 polypeptide. As disclosed herein, emergence or expansion of any one of these three lymphocyte populations is indicative of opportunistic disease. In yet other embodiments, the instant method involves determining the ratio of lymphocytes displaying a Vδ2 polypeptide or a Vγ9 polypeptide to those lacking a Vδ2 polypeptide or a Vγ9 polypeptide, respectively. Inversion of this ratio is believed to be indicative of infection by an opportunistic pathogen. In two currently preferred embodiments, these immunophenotypes can predict exposure to and/or infection by a nontuberculous mycobacterium, such as *Mycobacterium avium complex* (MAC) or a sporozoan parasite, such as *Toxoplasma gondii*.

As earlier described, the instant invention features a diagnostic method for determining the size of a population of T-lymphocytes displaying the CD3 antigen but lacking the CD4 and CD8 antigens. That is, T-cells having a CD3+/CD4-/CD8- immunophenotype can be determined. This subset of lymphocytes is also referred to herein and in the art as "double-negative" (DN) T-cells. As disclosed herein, emergence and/or expansion of this particular subset of T-lymphocytes is considered predictive of opportunistic disease. Certain other embodiments of the instant invention feature methods for determining emergence or expansion of two or more of the above-described populations of T-lymphocytes in a sample from an immunocompromised individual. For example, one embodiment involves determining the size of a population of lymphocytes displaying a CD3+/CD4-/CD8-/γδ+ immunophenotype. Another embodiment involves determining the size of lymphocyte populations displaying a CD3+/CD4-/CD8-/γδ+/ Vδ2- or CD3+/CD4-/CD8-/γδ+/Vγ9- immunophenotype. As described herein, emergence and/or expansion of lymphocytes displaying these particular phenotypes is indicative of opportunistic disease.

When practicing the instant invention, tissue samples containing lymphocytes are contacted with detectable agents that bind selectively to particular subsets of T-lymphocytes. The binding of these agents then permits detection of a particular T-lymphocyte population(s). In currently preferred embodiments, the methods rely on optical detection to determine emergence and/or expansion of certain T-lymphocyte populations, most preferably optical detection using fluorescent agents and flow cytometry methods. As contemplated herein, flow cytometry includes at least 2- and 3-color flow cytometry techniques well-known in the art. Specific flow cytometry protocols, and reagents suitable for use therewith, are set forth in detail below.

It is contemplated that detectable agents include, but are not limited to, those which can be used to detect T-lymphocytes displaying: γδ T cell receptors; γδ T cell receptors lacking a Vδ2 polypeptide or a Vγ9 polypeptide; a CD3+/CD4-/CD8- immunophenotype; a CD3+/CD4-/CD8-/γδ+ immunophenotype; a CD3+/CD4-/CD8-/γδ+/ Vδ2- immunophenotype; a CD3+/CD4-/CD8-/γδ+/Vγ9-immunophenotype; a γδ+/CD8+ immunophenotype; a Vδ2-/γδ+/CD8+ immunophenotype; and a Vγ9-/γδ+/CD8+ immunophenotype. As disclosed earlier, these agents can be used alone or in combination. These agents can be used to determine emergence and/or expansion of particular subsets of lymphocytes, as well as inversion of ratios of particular subsets of lymphocytes.

It is further contemplated that use of the above-described detectable agents can be made for the manufacture of kits for immunophenotyping lymphocytes according to each of the above-described methods. Accordingly, in another aspect, the invention provides diagnostic kits to facilitate practice of the instant methods. In one embodiment a kit contains an appropriate reference standard relative to which an alteration in the size of one or more of the preferred T-cell populations can be determined. As contemplated herein, kits for detecting a population emergence and/or expansion of T-cells displaying one of the following immunophenotypes are useful to predict opportunistic disease: CD3+/CD4-/CD8-; γδ+; Vδ2-; Vγ9-; γδ+/CD8+. As further contemplated herein, kits for detecting a population decline of T-cells displaying at least the following immunophenotypes also are useful to predict opportunistic disease: γδ+/Vδ2+; γδ+/Vγ9+; γδ+/CD8-.

Other kit embodiments permit monitoring and/or determining ratio inversions among certain populations of T-lymphocytes. The currently preferred population ratios useful for predicting opportunistic disease include, at least, the following: γδ+/CD8+ to γδ+/CD8- T-cells; γδ+/Vδ2- to γδ+/Vδ2+ T-cells; γδ+/Vγ9- to γδ+/Vγ9+ T-cells. Certain of these embodiments contain a suitable reference standard.

As disclosed earlier, preferred kit embodiments contain agents for optically detecting particular T-lymphocytes such as, but not limited to, those displaying: a γδ T cell receptor; a γδ T cell receptor lacking a Vδ2 polypeptide or a Vγ9 polypeptide; and, CD3 antigen but lacking CD4 and CD8 antigens. It is herein understood that optical detection includes detection by additive and/or subtractive methods as earlier explained. Other embodiments of the instant kits contain a panel of different agents which permit optical detection of multiple T-cell populations as also described above. All currently preferred kits rely on optical detection using fluorescent agents. As contemplated herein, detectable agents preferably are specific-binding proteins such as, but not limited to, antibodies. In some preferred embodiments, the binding agent further comprises a fluorophore such as, but not limited to, fluorescein (FITC), rhodamine, phycoerythrin (PE), phycoerythrin-sulfoindocyanine tandem conjugate (PE-Cy5), peridinin-chlorophyll (PER-CL) or Texas red. Suitable equivalents will be appreciated by the skilled artisan. In other embodiments, binding agents further comprise biotin, avidin, an enzyme, a radionuclide, a chromophore, and like moieties permitting detection of the binding agents contemplated herein.

Practice of the invention will be still more frilly understood from the following examples, which are presented herein for illustration only and should not be construed as limiting the invention in any way.

### EXAMPLE I: MATERIALS AND METHODS

### A. Patients and Their Clinical Classification

An exemplary immunocompromised individual from whom a tissue sample containing lymphocytes can be obtained is an HIV-seropositive patient. HIV-seropositive patients were evaluated for opportunistic diseases using the instant invention as described in Examples II and III set forth below. These patients were members of a defined clinical population known as the "Aquitaine" cohort. The "Aquitaine" cohort has been enrolled by the "Groupe d'Epidémiologie Clinique du SIDA en Aquitaine" (GESCA) from a surveillance program of HIV infection in the Bordeaux University Hospital and three secondary public hospitals in Aquitaine since 1987 (Chêne et al. (1992), 8 *Eur. J. Epidemiol.* 58-66 and Lasseur et al. (1995), 16 *Rev. Méd. Interne* 110-120), the disclosures of which are herein incorporated by reference).

For an eleven-month period, 498 HIV-1 positive patients with a CD4+ T-lymphocyte count of less than 100 per mm³ were followed up in this cohort. The staging of these patients was done according to the Center for Disease Control (CDC) (Atlanta, GA), classification of 1987. During this period, the immunological parameters of 381 patients, constituting the "study group," were serially determined in the reference routine Laboratory of Immunology of the Bordeaux University Hospital. The biological samples of the 117 other patients were processed in the local laboratories from the secondary public hospitals under different working protocols which did not allow the enumeration of the DN T-cells, and were therefore excluded from the present analysis. Fifty-seven age-matched healthy blood donors with no risk factor served as "control group." The general characteristics of the 381 patients, which are summarized in Table 1, are representative of the "Aquitaine" cohort patients who had been looked after for the same time period (N=498). For patients who remained free of AIDS-defining events during the entire follow-up period, the first determination of T-lymphocyte subpopulations was considered (n=216). For patients who suffered from one AIDS defining event, the concomitant or the closest determination (with a delay less than two weeks) to this episode was analyzed (n=166). Because of too small numbers, 5 oesophagal candidiases and 2 cryptosporidiosis infections were not considered. When two or three AIDS-defining events occurred successively in the same individual, and when the delay between the events was more than three months, one determination of T-lymphocyte subpopulations was considered for each episode and these were analyzed separately. When two or three AIDS-defining events were diagnosed at the same time or when the delay between the events was less than three months in one patient, only one T-lymphocyte determination was taken into account (n=17).

The diagnoses of diseases indicative of AIDS were made according to the criteria of the CDC (Center for Disease Control (1993), 41 *Morb. Mortal Wkly. Rep*. (RR-17):16-19, the disclosure of which is herein incorporated by reference).

**TABLE 1**

| Epidemiological characteristics of patients | | | | |
|---|---|---|---|---|
| **Population's Characteristics** | | **HIV Positive CD4+ Cells count < 100/mm**^{**3**} **present study (n=381)** | **HIV Positive CD4+ cells count < 100/mm**^{**3**} **"Aquitaine" cohort**^{**1**} **(n=498)** | **Normal Individuals HIV negative**^{**2**} **(n=57)** |
| Sex ratio M:F | | 4.3:1 | 3.8:1 | 1.1:1 |
| Age in 1994 | Median | 36 years | 36 years | 44.5 years |
| | Range | 15-83 | 10-92 | 21-63 |
| Risk Groups: | Homosexuals | 34.9% | 34.1% | NR⁴ |
| | IVDU³ | 31.8% | 29.5% | NR⁴ |
| | Homo+IVDU³ | 2.1% | 2.6% | NR⁴ |
| | Hemophiliacs | 1.0% | 3.0% | NR⁴ |
| | Heterosexuals | 12.1% | 13.1% | NR⁴ |
| | Blood Recipients | 7.9% | 6.4% | NR⁴ |
| | Indeterminate | 10.2% | 11.3% | NR⁴ |
| CDC Staging 1987 | II | 6.3% | 3.8% | NR⁴ |
| | III | 7.2% | 9.1% | NR⁴ |
| | IV non-AIDS | 23.4% | 25.9% | NR⁴ |
| | AIDS | 63.1% | 63.0% | NR⁴ |

| | | | | |
|---|---|---|---|---|
| 1. All patients seen during the study period. 117 of them were excluded from the present study because biological samples were not processed in the reference laboratory. | | | | |
| 2. Blood donors. | | | | |
| 3. Injection drug users. | | | | |
| 4. Not relevant because of blood donors at risk excluded. | | | | |

### B. Flow Cytometry: Reagents and Protocols

The immunophenotypes and size of T-cell populations can be determined using a variety of different flow cytometry protocols and a variety of different reagent configurations. Because the skilled artisan will appreciate the extent to which protocols and reagents can be varied, only a few representative or exemplary protocols and reagents are described herein in detail. In essence, practicing the instant invention requires only that certain preferred T-cell immunophenotypes be identified and their population size be determined. The precise step-by-step flow cytometry protocol, including the particular reagent(s) used to accomplish this, is not the essence of the discovery and invention disclosed herein.

### 1. General Guidelines

Immunophenotyping using flow cytometry is an art-recognized technique for identifying and/or isolating specific populations of lymphocytes, differentiated one from another by certain surface antigens and/or epitopes. As practiced herein, immunophenotyping methods were conducted in accordance with the performance guidelines established by the Centers for Disease Control (CDC), 1994 43 *Morb. Mortal. Wkly. Rep.* (RR-3):1-21, the disclosure of which is herein incorporated by reference. A brief summary of these guidelines follows; details not expressly presented below will be known to, and appreciated by, the skilled artisan.

For optimal results, the CDC recommends that immunophenotyping should be performed within 30 hours, but no later than 48 hours, after drawing the blood specimen. A direct two-or three-color immunofluorescence whole-blood lysis method is preferred, particularly preferred is the "stain, then lyse" procedure. Monoclonal antibody combinations should be used to enumerate CD4+ and CD8+ T-cells and to ensure the quality of the results. One exemplary two-color immunophenotyping antibody panel is in Table 1A, listed by CD nomenclature and fluorochrome; this panel (and an expanded version thereof) is recommended by the CDC in the above-referenced and incorporated guidelines. Results from this exemplary panel can provide data useful for: defining T-cell populations and subpopulations; determining the recovery and purity of the lymphocytes in the gate; accounting for all lymphocytes in the gate; setting cursors for positivity; and, monitoring the tube-to-tube variability.

**TABLE 1A**

| Recommended 2-color monoclonal antibody panel for lymphocyte immunophenotyping* | | |
|---|---|---|
| **FITC** | **PE** | **Reason for using** |
| CD45 | CD14 | To draw gates; lymphocytes are brightly positive for CD45 and negative for CD14 |
| Isotype | Isotype | To set cursors |
| CD3 | CD4 | To measure CD4+ T-cells; only cells positive for both CD4 and CD3 should be considered CD4+ T-cells |
| CD3 | CD8 | To measure CD8+ T-cells; only cells that are positive for both CD8 and CD3 should be considered CD8+ T-cells; the remainder of the CD8 cells (CD3-) are natural killer (NK)-cells. |

| | | |
|---|---|---|
| * Panel recommended by the CDC in : 43 *Morb. Mortal. Wkly. Rep*. (RR-3):1-21 (1994). | | |

As exemplified herein, three-color monoclonal antibody panels also can be used if the quality of immunophenotyping results from the three-color combinations can be assured and the panel has been validated using specimens from both HIV-infected and HIV-uninfected persons. Assurance of the results includes: a) validating the gating strategies used so that the quality of the gate is known (i.e., lymphocyte recovery and purity); and, b) a method for evaluating nonspecific fluorescence in the unlabeled population. As practiced herein, validation of a three-color panel includes labeling specimens with both the two-color panel and the proposed three-color panel, then determining whether the differences in results for a particular population (e.g., CD4+ T-cells) by both methods are within the variability expected from replicates in the laboratory.

When performing either 2- or 3-color flow cytometry, the CDC recommends premixed detectable binding agents, e.g., antibodies, at concentrations recommended by the manufacturer. If, instead, two or three single-color reagents are to be combined by the practitioner, each must be titered with the other(s) to determine optimal concentrations for use (e.g., 10 µL antibody A with 5 µL antibody B, 5 µL antibody A with 10µL antibody B, etc., for two-color; 10 µL antibody A with 5 µL antibody B and 5 µL antibody C, etc., for three-color). As will be obvious to the skilled artisan, reagents from different manufacturers are likely to be different in their epitope specificity, fluorochrome/protein (F/P) ratio, and protein concentrations. Because of these differences, combining reagents from different manufacturers is less preferred and should be done practicing routine care. For example, optimal antibody concentrations are those in which the brightest signal is achieved with the least amount of noise (i.e., the best signal-to-noise ratio). The nonspeciflc binding should be no greater than that of an isotype control. One way to evaluate the appropriate concentration of antibodies when combined is to evaluate the fluorescence histogram in a tube in which only one antibody is added and compare it with the histogram from a tube in which more than one antibody is added, the single-parameter histograms from both tubes should be similar. In addition, the percent positive cells for the cell population by both methods should be within the expected variability established in the laboratory.

When centrifuging cells mixed with binding agents, the CDC recommends that the practitioner maintain centrifugation forces of no greater than 400g for 3-5 minutes. Vortex sample tubes to mix the blood and reagents and break up cell aggregates. Vortex samples immediately before analysis to optimally disperse cells. Include a source of protein (e.g., fetal bovine serum or bovine serum albumin) in the wash buffer to reduce cell clumps and autofluorescence. Incubate all tubes in the dark during the immunophenotyping procedure.

Before analysis on the flow cytometer, the CDC advises that all samples be adequately fixed. Even though some of the commercial lysing/fixing reagents can inactivate cell-associated HIV, it is good laboratory practice to fix all tubes alter staining and lysing with 1%-2% buffered paraformaldehyde or formaldehyde. As is well-known to the skilled artisan, the characteristics of paraformaldehyde and formaldehyde may vary from lot to lot. They may also lose their effectiveness over time. Therefore, these fixatives should be made fresh weekly from electron microscopy-grade aqueous stock.

Immediately after processing the specimens, stained samples should be stored in the dark and at refrigerator temperatures (4-10° C) until flow cytometric analysis. Specimens should be stored for no longer than 24 hours unless the practitioner can show that scatter and fluorescence patters do not change for specimens stored longer.

With regard to negative and positive controls for immunophenotyping, a negative (isotype) reagent control should be used with each specimen to determine nonspecific binding of the mouse monoclonal antibody to the cells and to set markers for distinguishing fluorescence-negative and fluorescence-positive cell populations. For example, a monoclonal antibody with no specificity for human blood cells but of the same isotype(s) as the test reagents can be used. For positive methodologic controls, a suitable reagent is one useful to determine whether procedures for preparing and processing the specimens are optimal. This control should be prepared each time patient specimens are prepared. For example, a whole blood specimen from a control donor can be used. Ideally, this control will match the population of patients tested in the laboratory. If this control falls outside established normal ranges, the reason should be determined. The purpose of the methodologic control is to detect problems in preparing and processing the specimens. For positive controls for testing reagents, a suitable reagent is one useful to test the labeling efficiency of new lots of reagents or when the labeling efficiency of the current lot is questioned. For example, a whole blood specimen or other human lymphocyte preparation (cryopreserved or lyophilized lymphocytes) can be used.

As will be appreciated by the skilled practitioner, flow cytometer quality control requires the following: aligning optics daily; standardizing daily; determining fluorescence resolution daily; compensating for spectral overlap daily; repeating all four instrument quality control procedures whenever instrument problems occur or if the instrument is serviced during the day; maintaining instrument quality control logs.

Data analysis is discussed below in more detail, however, general recommendations from the CDC include the following: Draw lymphocyte gates using forward and side light-scattering patterns and fluorescence staining. Other art-recognized methods of drawing lymphocyte gates can be used with three-color monoclonal antibody panels. Verify the lymphocyte gate by determining the recovery of lymphocytes within the gate and the lymphocyte purity of the gate. As used herein, the lymphocyte recovery (also referred to as the proportion of lymphocytes within the gate) is the percentage of lymphocytes in the sample that are within the gate. The lymphocyte purity of the gate is the percentage of cells within the gate that are lymphocytes. The remainder may be monocytes, granulocytes, red cells, platelets, and debris. The lymphocyte recovery should be optimally be at least about 95%. The lymphocyte purity of the gate should optimally be at least about 90%. Optimal gates include as many lymphocytes and as few contaminants as practicable. As practiced herein, lymphocyte recovery within the gate using CD45 and CD14 can be determined by two different methods: light scatter gating and fluorescence gating. The number of lymphocytes identified will be the same whether determined by light scatter gating or by fluorescence gating.

### 2. Exemplary Protocols and Reagents Suitable for Use With the Present Invention

### (a) Exemplary Protocol I

Viable lymphocytes can be isolated by ficoll-hypaque density centriftigation and contacted with a detectable specific binding agent such as a specific monoclonal antibody available commercially, e.g., OKT®3, OKT®4 or OKT®8 (Ortho Diagnostic Systems, Inc., Raritan NJ), for 30 minutes at 4° C. OKT® is a registered trademark of Ortho Diagnostics, Inc.; the OKT designation number corresponds to the art-recognized T-cell classification terminology for "cluster designation" or "CD." Thus, OKT®3, 4 and 8 bind selectively to CD3, CD4 and CD8, respectively. CD3, CD4 and CD8 specific binding agents are available from numerous other suppliers well-known to the skilled practitioner. After washing, the cell pellets can be contacted further with detectable fluorescein isothiocyanate (FITC) conjugated goat anti-mouse IgF(ab)'₂ fragments. Flow cytometry can then be performed on an Ortho® cytofluorograph (Ortho Diagnostic Systems, Inc., Westwood MA) or a Coulter Epics cytometer (Coulter Electronics, Hyaleah FL) using routine procedures previously described by the CDC, for example. Specifically stained positive cells can be determined relative to a negative control profile for each cell line (stained with a nonspecific control monoclonal antibody). Skilled artisans will appreciate that cells having fluorescence intensity channel numbers greater than the intercept of the negative control profile with the baseline are counted as positive, and the % positive is calculated relative to the total number of cells counted.

For certain embodiments of the instant invention, two-color flow cytometry analysis of normal adult peripheral blood lymphocytes can be performed using an anti-TCR γδ monoclonal antibody and an anti-CD3 monoclonal antibody. In some embodiments, peripheral blood lymphocytes can be stained first with an FITC-conjugated anti-CD3 monoclonal antibody (e.g., OKT®3) and then with a biotinyl-anti-TCR γδ monoclonal antibody followed by phycoerythrin-conjugated avidin.

### (b) Exemplary Protocol II

To measure T-cell subsets, specimens of heparinized whole blood can be stained with monoclonal antibodies using the well-known whole blood method. Briefly, particular antibodies conjugated to either fluorescein isothiocyanate (FITC) or phycoerythrin (PE) are added and, alter staining for 20 min on ice and 10 min at room temperature, the erythrocytes are lysed using an ammonium chloride-based lysing solution (Becton-Dickinson, Mountainview CA). The stained lymphocytes are fixed in 1% paraformaldehyde and proportions of stained cells are quantitated using a flow cytometer such as the EPICS C (Coulter Electronics, Hialeah FL).

Briefly, lymphocytes can be gated based on either fluorescence or on forward and 90° light scatter on linear scales. In some embodiments, five thousand cells are counted for each antibody combination. The percentage of positive cells for each marker or combination of markers is determined routinely after subtracting the background obtained with an appropriately conjugated isotype control antibody as described above. Absolute numbers (cells/mm³) of lymphocyte subsets are calculated by multiplying the percentage of positive cells for a given subset by the number of lymphocytes per mm³ in the peripheral blood, as determined from a complete blood count. Several types of controls can be used for assessing background and establishing fluorescence cutoffs. For example, nonspecific staining is detected with a combination of IgG₁-FITC and IgG₁-PE. As described above, in order to assure the accuracy of fluorescence measurements in two-color preparations, fluorescence cutoffs are checked using each FITC- and PE-conjugated antibody by itself, thereby setting the color compensation. As described below, contamination of the lymphocyte population with unlysed erythrocytes and with neutrophils and monocytes can be assessed using both CD45 conjugated with FITC and CD14 conjugated with PE. Specimens typically contain at least about 95% lymphocytes (brightly positive for CD45 and negative for CD14) and approximately less than 2% monocytes (brightly positive for both CD45 and CD14).

### (c) Other Detectable Agents Suitable For Use With The Present Invention

Certain embodiments of the present invention rely on T-cell immunophenotyping using 3-color flow cytometry. Three-color flow cytometry is practiced preferably using three fluorescent moieties having different emission spectra yet having similar excitation spectra. For example, it is preferred currently that each fluorescent moiety be excitable at approximately 488 nm yet have an emission spectrum substantially distinct from the others, permitting satisfactory resolution and detection by standard flow cytometry techniques using standard flow cytometer apparatus. One possible configuration of reagents can utilize the fluorescent moieties fluorescein (FITC), phycoerythrin (PE), and an art-recognized tandem moiety comprising phycoerythrin and a sulfoindocyanine dye such as, for example, PE-Cy5. While each of these fluorescent moieties is excitable with an argon-ion laser at 488 nm, their peak emissions are spectrally distinct, i.e., FITC emits at approximately 520 nm, PE emits at approximately about 576 nm and PE-Cy5 emits at approximately about 670 nm.

Particularly useful fluorochromes are the phycobiliprotein fluorochromes such as phycoerythrin, pllycocyanin, and allophycocyanin for multi-parameter immunoflourescence analyses. Phycobiliproteins are well-suited for multiparameter fluorescent analyses because of their strong absorption bands starting at 440 nm, their intense emission bands which extend into the far red spectrum, and their large Stokes shift relative to more conventional fluorescent moieties like FITC. As will be appreciated by the skilled artisan, phycofluors have been used extensively in fluorescence-activated cell sorting for cell surface analyses and can be adapted for two-, three-, and four-color quantitative analyses of markers on the surface of lymphocytes involving both single- and multi-laser excitation techniques. It is contemplated that the instant invention can be practiced using any one or more of these fluorescent moieties and 2-, 3- or 4-color flow cytometry.

As earlier described, tandem phycofluors permit three-color analyses with a single laser because tandem conjugates, such as phycoerythrin covalently linked to a sulfoindocyanine dye such as Cy5, exhibit a very large separation between absorption and emission wavelengths. For example, while Cy5 alone is excitable at approximately 650 nm, Cy5 tandemly conjugated to phycoerythrin is excitable at 488 nm. That is, phycoerythrin acts as an energy donor and transfers excitation energy to Cy5 so that the tandem conjugate actually emits at approximately a peak of about 670 nm, i.e., not within the phycoerythrin spectrum. Taken together with the fact that phycoerythrin alone emits at approximately a peak of 575 nm, the combined use of a phycoerythrin phycofluor and a phycoerythrin Cy5 tandem phycofluor can result in substantial spectral separation for fluorescent cell-sorting applications. Use of a third fluorescent moiety, such as fluorescein which is also excitable at 488 nm yet emits at a third emission spectrum of approximately 520 nm, permits single-excitation, 3-color immunofluorescence of the sort contemplated herein. The above-described fluorescent moieties conjugated with specific binding agents such as, for example, T-cell-specific antibodies, are available from Immunotech (France).

### (d) Exemplary Protocol III

For the patient studies in Examples II and III set forth below, TCR-specific monoclonal antibodies were obtained from Immunotech (France); all other monoclonal antibodies were purchased from Becton-Dickinson Co. (Mountainview CA). Cells were analyzed by two- or three-color flow cytometry following the manufacturer and the CDC recommendations (Center for Disease Control (1994) 43 *Morb. Mortal Wkly. Rep.* (RR-3): 1-21, previously incorporated by reference herein). All routine peripheral blood samples were stained in two separate tubes with either anti-CD14-Phycoerythrin (PE)/anti-CD45-Fluorescein isothyocyanate (FITC) and with anti-CD4-FITC/anti-CD8-PE/anti-CD3-Peridinin-Chlorophyll (Per-CP) monoclonal antibodies. The first labeling was done to ensure that only the population of lymphocytes (CD45+CD14-) gated on the basis of the forward angle and side scatter was analyzed, while the second analysis allowed the enumeration of every CD3+ T-lymphocyte subpopulation on a CD4/CD8 plot. Analysis of TCR expression by double negative (DN) T-cells i.e. T-cells having the immunophenotype (CD3+, CD4-, CD8-) was performed by flow cytometry using FITC-coupled CD4 and CD8-specific monoclonal antibody and either an anti-TCR αβ-PE or an anti-TCR γδ-PE monoclonal antibody. As a control, anti-CD3-PerCP was used instead of anti-TCR monoclonal antibody in a third reaction tube. Isotypic controls (IgG1-PerCP, IgG1-FITC, IgG1-PE, and IgG1-FITC/IgG2a-PE) also were used to check for nonspecific binding of antibodies and routinely were found negative.

In all cases, at least 5,000 lymphocytes were used for percentage determinations. Distributions of immunological parameters were described by their median value, first and third quartiles. First, the distribution of CD4, CD8 and DN T-cell percentages between groups of patients were compared using non-parametric Mann-Whitney U test, with a level of significance of 0.05. When parameters other than CD4+ T-cell percentage were found associated with AIDS-defining illnesses in the univariate analysis, a logistic regression model (BMDP Statistical Softwares, Los Angeles CA) was used to delineate the respective contribution of each immunological variable in predicting occurrence of AIDS-defining events. The magnitude of the association was estimated by the odds ratios (OR) with their 95% confidence limits (CI). The various statistical analyses presented herein were conducted using art-recognized parameters and theory.

### EXAMPLE II: CD4-CD8- DOUBLE NEGATIVE T-CELLS ARE PREDICTIVE OF DISSEMINATED MAC INFECTION, TOXOPLASMOSIS AND KAPOSI'S SARCOMA

### A. Identification of T-cell subsets (CD4+, CD8+, and DN) in control vs. patient groups

As earlier described, patients from the "Aquitaine" cohort were the clinical subjects evaluated in Examples II and III set forth herein. The cell surface antigen expression pattern of peripheral blood T-cells from HIV seropositive patients was examined and compared to a group of 57 healthy blood donors. All patients were lymphopenic with a median total lymphocyte count of 704 vs. 1830 per mm³ for the control group (Table 2). HIV patients had normal percentages of CD3+ T-cells, lower percentages of CD4+ and higher percentages of CD8+ T-cells. Considering the medians in both groups, the percentages of DN T-cells were at first glance elevated, but the range of observed values was much wider in the patient's group, with some values exceeding 10%. Analysis of the absolute number of cells instead of their percentages yielded similar conclusions. Analysis of the percentage of DN T-cells, however, allowed a better discrimination of an amplification of the DN T-cell subset, especially when patients were highly lymphopenic.

**TABLE 2**

| Immunological parameters of patients in the study | | | | |
|---|---|---|---|---|
| **Immunological parameters** | **Normal Individuals (n=57) (57 determinations)** | | **HIV+ patients with CD4+ cells counts - <100/mm**^{**3**} **(n=381) (399 determinations)** | |
| | median | quartiles | median | quartiles |
| Total lymphocytes count¹ | 1830 | 1556-2222 | 704 | 430-1030 |
| % of CD3+ cells² | 74.4 | 67.9-80.9 | 71.3 | 59.2-80.7 |
| % of CD4+ cells² | 41.7 | 36.3-48.0 | 4.00 | 1.31-7.17 |
| % of CD8+ cells² | 27.3 | 21.2-31.8 | 60.0 | 48.6-70.5 |
| % of double negative cells² | 2.1 | 1.4-3.4 | 3.30 | 2.00-5.55 |

| | | | | |
|---|---|---|---|---|
| 1. Automated blood cell counts were performed with an H2 Technicon device (Bayer, Germany). | | | | |
| 2. Fifty µl of whole blood were incubated at 4°C with 10 µl of MoAb. After 30 min., 2 ml of 10 times diluted FACS lysine solution (Becton-Dickinson, Mountainview, CA) were added and left 10 min. at room temperature. White blood cells were then spun down, washed once with PBS, and resuspended in 200 µl of PBS before flow cytometry analysis on a FACScan apparatus (Becton-Dickinson). | | | | |

### B. Univariate statistical analysis

The fact that the range of DN T-cell percentages was wider in patients suggested its heterogeneity. A search for clinical correlates of this immunological variable was then undertaken as follows. Patients were stratified, based on the documented illness they presented closest to the date of blood examination (see above discussion). Nine groups were defined, gathering patients with tuberculosis (15/399 determinations), MAC infections (40/381 determinations), toxoplasmosis (34/381 determinations), Kaposi sarcoma (39/381 determinations), pneumocystosis (19/381 determinations), lymphoma (10/381 determinations), CMV infections (9/381 determinations), associated illnesses (17/381 determinations) and asymptomatic patients (216/381 determinations). Again, the percentages of CD4+, CD8+ or DN T-cells were examined by comparing each group to a reference group of 216 asymptomatic patients (Table 3). Using art-recognized statistical methods, the non-parametric Mann-Whitney U test was used to evaluate data because the high variances observed in some groups precluded a univariate statistical analysis based on the percentage values.

**TABLE 3**

| Comparison of immunological parameters according to AIDS defining illnesses (Univariate analysis) | | | | | | | |
|---|---|---|---|---|---|---|---|
| **AIDS defining illness**^{**2**} | **n** | **% of CD4+ cells** | | **% of CD8+ cells** | | **% of DN cells** | |
| | | median (quartiles) | p¹ | median (quartiles) | p¹ | median (quartiles) | p¹ |
| Tuberculosis² | 15 | 4.90 (1.75-6.80) | 0.32 | 59.9 (35.2-71.5) | 0.68 | 3.55 (1.45-4.90) | 0.68 |
| MAC infection³ | 40 | 1.35 (0.35-3.12) | <10⁻⁴ | 52.0 (38.3-65.3) | 8x10⁻³ | 7.30 (4.11-9.32) | <10⁻⁴ |
| Toxoplasmosis⁴ | 34 | 1.45 (0.44-3.76) | <10⁻⁴ | 52.8 (41.9-69.6) | 0.11 | 4.57 (2.69-6.86) | 10⁻⁴ |
| Kaposi sarcoma⁵ | 39 | 3.75 (0.90-5.05) | 4x10⁻⁴ | 60.7 (55.6-74.2) | 0.50 | 4.30 (2.30-7.35) | 2x10⁻⁴ |
| Pneumocystosis⁶ | 19 | 3.9 (1.20-4.25) | 6x10⁻³ | 59.8 (56.9-72.5) | 0.63 | 2.80 (1.50-4.10) | 0.99 |
| Lymphoma⁷ | 10 | 6.77 (1.47-9.22) | 0.76 | 61.1 (43.0-68.2) | 0.45 | 3.16 (1.76-3.72) | 0.97 |
| CMV infection⁸ | 9 | 0.65 (0.37-2.21) | 6x10⁻⁴ | 55.7 (26.5-71.0) | 0.19 | 3.70 (1.72-8.02) | 0.12 |
| Associated Illnesses⁹ | 17 | 1.72 (0.30-5.21) | 7x10⁻⁴ | 55.8 (50.8-66.8) | 0.29 | 4.10 (2.35-7.39) | 7x10⁻³ |
| None | 216 | 5.30 (2.78-8.91) | | 63.18 (50.1-71.4) | | 2.68 (1.70-4.09) | |

| | | | | | | | |
|---|---|---|---|---|---|---|---|
| 1. Comparison using the no illness category as the reference group, Mann-Whitney U test. | | | | | | | |
| 2. Tuberculosis (TB) was defined by the presence of symptoms consistent with TB, associated with a positively confirmed *Mycobacterium tuberculosis* culture. | | | | | | | |
| 3. Disseminated *Mycobacterium avium* complex (MAC) infection was defined by the positivity of either blood, bone marrow or hepatic tissue specimen culture on adequate broth (BBL-Septi-chek, Becton-Dickinson, Mountainview, CA). | | | | | | | |
| 4. Toxoplasmosis of brain, suggested by recent onset of a neurological abnormality consistent with an intracranial process or a reduced level of consciousness, was confirmed by a typical picture at brain imaging, a positive titer of serum antibody to *Toxoplasmosa gondii* and a successful response to a specific therapy. | | | | | | | |
| 5. Presumptive diagnosis of Kaposi sarcoma was suggested by a characteristic appearance of an erythemateous or violaceous placque-like lesion on skin or on mucous membrane (in this case biopsy was not systematically performed to confirm the diagnosis). | | | | | | | |
| 6. *Pneumocystic carinii* pneumonia (PCP) diagnosis was established upon microscopic examination of bronchoalveolar lavage. | | | | | | | |
| 7. Lymphoma diagnosis was made by microscopic examination and appropriate immunocytochemical staining. | | | | | | | |
| 8. Presumptive cytomegalovirus (CMV) retinitis, which was the only CMV infection type which we had to deal with in this study, was ascertained by serial opthalmoscopic examinations. | | | | | | | |
| 9. MAC+KS (n=7), MAC+CMV (n=2), MAC+Toxo (n=2), MAC+CMV (n=1), MAC+KS (n=1), KS+CMV (n=2), KS+Toxo+Lymphoma (n=1), and KS+PCP (n=1). | | | | | | | |

The results of these patient assessments were as follows. The CD4+ T-cells subset was significantly diminished in all groups except tuberculosis and lymphoma-bearing patients. MAC disseminated infections, toxoplasmosis, and CMV infections were associated with the most severe depletion of CD4+ T-cells. In contrast, a significantly increased percentage of DN T-cells was only seen in patients with MAC infections, toxoplasmosis, Kaposi sarcomas and associated illnesses including at least one of these pathologies.

A graphical representation of this situation is provided in the accompanying Figure. In the Figure, the percentages of CD4+ are plotted against the percentages of DN T-cells for patients expressing a given pathology (closed circles) vs. asymptomatic patients (open circles). Although the size of the samples were not comparable, no correlations were evidenced between these two variables, Suggesting their relative independence. In agreement with the above-described univariate analysis, patients with MAC infections (panel A), toxoplasmosis (panel B), Kaposi sarcoma (panel C) or CMV infections (panel H) expressed higher percentages of DN T-cells. This is most obvious for the MAC group, especially in the HIV-seropositive individuals with CD4+ T-cells below 50/mm³ or 7%, who were at high risk of these infections. In contrast, none of the other illnesses herein studied, particularly tuberculosis, exhibited this specific pattern. Patients with CMV infections had a more drastic CD4+ T-cell lymphopenia; however, although the range of DN T-cell percentages in the CMV group was comparable to that of Kaposi's sarcoma patients, it was not significantly different from that of the reference group, perhaps due to the limited size of the sample (n=9).

### C. Multivariate logistic regression:

Based on the above-described univariate analysis, a multivariate logistic regression was performed in an attempt to identify predictors of occurrence of opportunistic disease particularly MAC disseminated infections and toxoplasmosis. The DN T-cell percentage was an independent and strong predictor of MAC infections, generally as powerful as the CD4+ T-cell percentage (Table 4). A significant statistical association was also observed for toxoplasmosis and Kaposi's sarcoma patients, but the predictive value was weaker for DN T-cell than for CD4+ T-cell percentages.

**TABLE 4**

| Multivariate logistic regression of MAC infections, toxoplasmosis, Kaposi sarcomas and the percentages of CD4+, CD8+ and DN T-cells | | | |
|---|---|---|---|
| **ODDS RATIO OR (95%CI) p value** | **%CD4**^{**1**} | **%DN**^{**2**} | **%CD8**^{**1**} |
| MAC³ | 5.3 (3.3-8.4) p<10⁻³ | 5.0 (3.5-7.2) p<10⁻⁵ | 1.1 (1.0-1.2) p=0.10 |
| Toxoplasmosis³ | 4.0 (2.7-5.9) p<10⁻³ | 2.4 (1.7-3.4) p<0.01 | 1.0 (1.0-1.1) p=0.44 |
| Kaposi's Sarcoma³ | 2.6 (1.9-3.5) p<0.01 | 2.4 (1.8-3.3) p<0.01 | 1.1 (1.0-1.2) p=0.14 |

| | | | |
|---|---|---|---|
| 1. for a decrease of 5% | | | |
| 2. for an increase of 5% | | | |
| 3. against the no AIDS-defining events group during the study period. | | | |

As expected from the above-described graphical representation, a similar analysis of a patient group with a lower CD4+ T-cell cut-off (i.e., below 50 CD4+ T-cells per mm³) indicated that the odds ratio for prediction of MAC infections was decreased to 2.5 (1.5-4.2) relative to the percentage of CD4+ T-cells, but the predictive value remained high for the percentage of DN T-cells with a point estimate of 5.3 (3.6-7.8). Decreasing further the CD4+ T-cell cut-off did not improve the predictive correlation.

In summary, these data indicate that the percentages of DN and CD4+ T-cells fluctuate in an independent manner in HIV-seropositive patients. Moreover, at low CD4+ cell counts, when the patients are at an especially high risk for opportunistic infections (i.e., CD4+ T-cells below 50 cells per mm³), the percentage of DN T-cells was significantly increased in patients with MAC disseminated infections, providing a previously unappreciated predictor of this disease.

### D. The Predictive Value of Double-Negative T-Cells for MAC, Toxoplasmosis and Kaposi's Sarcoma but not Classical Tuberculosis

In an effort to extend the above-described studies, the TCR subtype expressed by the DN T-cells was determined by flow cytometry using TCR γδ and αβ chain-specific monoclonal antibodies on 75 randomly chosen patients from the study group. The large majority of the above-described DN T-cells expressed γδ TCR (7.8% +/- 3.9 of DN T-cells versus 5.7% +/- 4.2 of γδ T-cells (% +/- SD)). Furthermore, there was a detectable expansion of the T-cell subset displaying the γδ immunophenotype.

In addition to confirming an association between a decreased percentage of CD4+ T-cells in HIV patients and the occurrence of AIDS-defining events, these data demonstrate quantitatively-specific enlargement of the DN γδ T-cell subset in HIV-seropositive individuals. Of particular significance is the fact that this expansion correlates with the onset at least of MAC disseminated infections and toxoplasmosis. Of further significance is the observation that patients with classical tuberculosis or pneumocystosis were in the normal range. These data, together with the fact that only some diseases led to a specific increase in γδ T-cells, strongly suggest that the expansion was antigen-driven. It is especially relevant clinically that classical tuberculous mycobacterial infection did not correlate with a quantitatively specific expansion of DN T-cells. Thus, as described herein, a predisposition to disseminated MAC infection can be distinguished clinically from classical tuberculosis by practicing the instant invention.

Based on earlier reports, this observation was entirely unexpected. For example, studies describing *in vivo* and *in vitro* activation of γδ cells by tuberculous mycobacteria (Ladel et al. (1995) 25 *Eur. J. Immunol*. 838-846; Kronenberg (1994) 6 *Curr. Opin. Immunol*. 64-71) indicated that some expansion of γδ T-cells should occur in HIV patients with classical tuberculosis. It was unexpected, therefore, that the percentage of γδ T-cells would remain in the normal range in HIV seropositive patients infected with classical tuberculous mycobacteria, *Mycobacterium tuberculosis*. Other reports attempted to implicate γδ T-cells in parasitic infections (Haas et al. (1993) 11 *Ann. Rev. Immunol*. 637-685; Kronenberg (1994) 6 *Curr. Opin. Immunol*. 64-71); however, no definitive correlation was possible. Thus, the presently observed correlation between an expanded population of γδ T-cells with toxoplasmosis was unexpected.

Also of interest, the above-described multivariate logistic regression analysis confirmed the independence of fluctuations of DN and CD4+ T-cell percentages, since both are excellent predictors of MAC infections. Therefore, these data also indicate that the percentages of DN T-cells represent a valuable additional marker to be taken into account in predictive models leading to clinical decisions for management of MAC.

In conclusion, the above-described study has identified the percentage of DN T-cells as a predictor at least for the presence of MAC infections or toxoplasmosis. Clinically, a differential diagnosis can now be made which renders this new parameter of immense value in predicting early onset of MAC disseminated infections in HIV patients.

### EXAMPLE III: T-CELL RECEPTOR SUBTYPES WITHIN DN T-CELLS

### A. Activated DN T-cells and Their Predictive Value

It is known that γδ T-cells can express also a homodimeric CD8 αα coreceptor, thought to be associated with activation of these cells. In one specific study, the proportion of CD8+ cells among γδ T-cells was increased when the percentage of DN T-cells was above 6%, which indicates that a quantitative increase of this cell subset is accompanied by its activation.

It is anticipated that a more thorough analysis of the percentage of total γδ T-cells and a determination of the ratio between resting versus activated γδ T-cells will further confirm that such cells have clinically-significant predictive value. Accordingly, the ratio of γδ+/CD8+ to γδ+/CD8- T-cells will be determined and/or monitored in certain HIV-seropositive patients. It is expected that opportunistic disease will correlate with an emergence and/or expansion of the activated immunophenotype γδ+/CD8+, while the unactivated immunophenotype γδ+/CD8-will either remain unchanged and/or decline. It is further expected that the ratio of γδ+/CD8- to γδ+/CD8+ will invert, such that a prevalence of activated γδ+ cells is predictive of opportunistic disease, especially MAC infection or toxoplasmosis.

### B. T-Cell Receptor Epitope Expression in DN T-cells and its Predictive Value

As will be appreciated by the skilled artisan, most peripheral blood γδ T-cells in immunocompetent individuals express a particular combination of variable (V) region gene products (Vγ9 and Vδ2) to form their TCR (Triebel et al. (1988) 18 *Eur. J. Immunol.* 2021-2027; Casorati et al. (1989) 170 *J. Exp. Med.* 1521-1535; Parker et al. (1990) 171 *J. Exp. Med.* 1597-1612). Antigen-driven selection and expansion of such γδ T-cells should, however, not only lead to a quantitative increase of this phenotype, but also to qualitative differences in V region expression within this phenotype.

In this regard, the above-described patient study has led to the discovery that a γδ+ T-cell increase usually is accompanied by a strong decline in the Vδ2 subpopulation and a predominance of γδ T-cells lacking the Vδ2 epitope. Moreover, a ratio inversion also was discovered in at least one patient with a confirmed MAC disseminated infection, but an apparently normal percentage of DN T-cells. Thus it is anticipated that qualitative alterations among at least the Vδ2 and Vγ9 subsets of γδ+ T-cells will be predictive of disease in patients who suffer from MAC infection, toxoplasmosis and similar opportunistic diseases. It further is anticipated that a predictive hierarchy of immunophenotypes including at least these immunophenotypic parameters can be constructed which will permit progressively confirmatory diagnoses.

### C. Prospective Studies of DN T-cells as Predictors of Opportunistic Diseases

Studies are in progress to confirm and extend the above-described discovery that certain subtypes of DN T-cells are predictive of MAC, toxoplasmosis and similar conditions. Using the instant invention, characterization of at least the following T-cell populations is expected to predict specific opportunistic disease(s):
1. Samples of peripheral blood lymphocytes from certain immunocompromised individuals will contain expanded populations of γδ T-cells lacking the Vδ2 or the Vγ9 epitope; other samples will have a concomitant decline in the population of γδ T-cells displaying the Vδ2 or the Vγ9 epitope. Moreover, an inversion of the ratio of T-cells expressing the Vδ2+ to Vδ2- or the Vγ9+ to Vγ9- immunophenotypes, relative to a reference standard, should be observed in certain immunocompromised individuals. It is expected that such perturbations in T-cell populations will correlate with exposure to, onset or progression of, and/or relapse from infection with MAC and/or *T. gondii*. Furthermore, it is expected that ratio values which specifically predict the incidence of each of these diseases will be calculated.
2. Samples of peripheral blood lymphocytes from certain immunocompromised individuals will contain a population of γδ T-cells co-expressing the CD8 antigen. It is expected that the ratio of γδ+/CD8+ to γδ+/CD8- phenotypes will correlate with disease. That is, relative to a suitable reference standard, the population of γδ+/CD8+ will be expanded thereby causing an increase in the ratio of γδ+/CD8+ to γδ+/CD8-. It is expected that such perturbations in T-cell populations will correlate with exposure to, onset or progression of, and/or relapse from infection with MAC and/or *T. gondii*. Because the expression of CD8 among γδ+ cells is indicative of T-cell activation, it is further expected that any detectable perturbation in the ratio of activated to non-activated γδ+ cells should be indicative of opportunistic diseases. Furthermore, it is expected that ratio values which specifically predict the incidence of each of these diseases will be calculated.

Generally speaking, the studies in progress will be conducted using the protocols and reagents disclosed herein or routine modification thereof. Patients will be members of the aforementioned Acquitaine cohort, although results obtained with this cohort are expected to be of far more general applicability to immunocompromised patients. The number of patients will be approximately 200. Patient samples will be collected at prescribed intervals and patients will be actively monitored for approximately six months and thereafter undergo regular clinical follow-up for approximately 12 more months. Patients seropositive for HIV-1 and HIV-2 will be studied. Only patients at least 20 years of age and having less than 100 CD4+/mm³ will be included; patients also must have a life expectancy of at least 6 months. Prior to commencement of the study, patients will be evaluated clinically for those pathologies known to affect the size of either DN T-cells or γδ+ T-cells. Specifically, patients will be tested for infection by MAC, *T. gondii*, visceral leishmaniasis and malaria. Positive diagnosis of any of these pathologies (using conventional clinical methods as earlier described) will not cause a patient to be excluded, rather it will be used as a stratification variable. Correlations between clinical parameters and T-cell immunophenotypes will be determined using univariate and multivariate data analyses as disclosed herein above. Data analyses will be facilitated by using BMPD Software (BMPD Statistical Software, Inc., Los Angeles CA).

### Equivalents

The invention may be embodied in other specific forms without departing from the spirit or essential characteristics thereof. The foregoing embodiments are therefore to be considered in all respects illustrative rather than limiting on the invention described herein. Scope of the invention thus is indicated by the appended claims rather than by the foregoing description, and all changes within the meaning and range of equivalency of the claims are intended to be embraced therein.

## Claims

1. A method for assessing opportunistic disease in an immunocompromised mammal, comprising the step of:
determining, in a tissue sample obtained from said mammal, the size of a population of lymphocytes displaying a γδ T-cell receptor, said γδ T-cell receptor optionally being free of a polypeptide selected from Vδ2 or Vγ9,
wherein expansion of said population, relative to a reference standard, provides information that treatment of said immunocompromised mammal may be warranted to control opportunistic disease associated with infection with a nontuberculous mycobacterium or a sporozoan parasite.

2. A method for assessing opportunistic disease in an immunocompromised mammal, comprising the step of:
determining, in a tissue sample obtained from said mammal, the size of a population of lymphocytes displaying a CD3+CD4-CD8-phenotype,
wherein expansion of said population, relative to a reference standard, provides information that treatment of said immunocompromised mammal may be warranted to control opportunistic disease associated with infection with a nontuberculous mycobacterium or a sporozoan parasite.

3. The method of claim 2 further comprising the step of determining, in said tissue sample, the size of a population of lylnphocytes displaying a CD3+/CD4-/CD8-/αβ+ phenotype.

4. A method for assessing exposure of an immunocompromised mammal to an opportunistic intracellular pathogen selected from a nontuberculous mycobacterium or a sporozoan parasite, comprising the steps of:
(a) providing a tissue sample obtained from said mammal and comprising lymphocytes;
(b) contacting said sample with a detectable first agent that binds selectively to T-lymphocytes displaying a γδ T-cell receptor; and,
(c) detecting lymphocytes bound selectively by said first agent in said sample,
wherein expansion of a population of said detected lymphocytes, relative to a population thereof in an immunocompromised mammal free of said pathogen, provides information that treatment of said immunocompromised mammal may be warranted to control infection by said pathogen.

5. The method of claim 1, 2 or 4 wherein (i) said nontuberculous mycobacterium is *Mycobacterium avium complex* (MAC), *M. avium intracellulare, M. kansasii, M. scrofulaceum, M. ulcerans, M. marinum, M. xenopi, M. szulgai, M. simiae, M. hemophilium, M. genovensee, M. fortuitum*, or *M. chelonei;* or wherein (ii) said sporozoan parasite belongs to genus *Leishmania, Isospora, Toxoplasma, Plasmodium, Babesia, Cryptosporidium* or *Sarcocystis*; or wherein (iii) said sporozoan parasite is *Toxoplasma gondii*.

6. The method of claim 1, 2 or 4 further comprising monitoring said population of detected lymphocytes over time by repeating steps (a)-(c), wherein said steps are repeated at least once using at least two different samples obtained at two different times from said mammal; or wherein said monitoring is performed until said expansion is detectable.

7. The method of claim 1, 2 or 4 wherein (i) said first agent is detectable optically; or wherein (ii) detection is accomplished by flow cytometry.

8. The method of claim 1, 2 or 4 wherein said detectable first agent is a specific-binding protein.

9. The method of claim 1, 2 or 4 wherein said immunocompromised mammal is a primate, optionally afflicted with an immune deficiency syndrome.

10. The method of claim 9 wherein said primate is a human afflicted with AIDS, further wherein said human fails to exhibit symptoms normally attributable to either of said pathogens.

11. The method of claim 4 further comprising the steps of:
(d) contacting said sample with a detectable second agent that binds selectively to T-lymphocytes displaying CD8; and,
(e) detecting, in said sample, lymphocytes displaying a CD8+/γδ+ immunophenotype,
said method optionally still further comprising the steps of:
(f) contacting said sample with a detectable third agent that binds selectively to T-lymphocytes displaying a γδ T cell receptor comprising a Vδ2 polypeptide or a Vγ9 polypeptide; and,
(g) detecting, in said sample, lymphocytes displaying a CD8+/γδ+, a CD8+/Vδ2-, or a CD8+/Vγ9- immunophenotype,
wherein expansion of a population of lymphocytes displaying any of said immunophenotypes, relative to a population thereof in an immunocompromised mammal free of either of said pathogens, provides information that treatment of said immunocompromised mammal may be warranted, to control infection by at least one of said pathogens.

12. The method of claim 4 further comprising the steps of:
(d) contacting said sample with a detectable second agent that binds selectively to T-lymphocytes displaying a γδ T-cell receptor comprising a Vδ2 polypeptide or a Vγ9 polypeptide; and,
(e) detecting, in said sample, lymphocytes displaying a γδ+/Vδ2+ or a γδ+/Vγ9+ immunophenotype,
wherein a decline in said γδ+/Vδ2+ or said γδ+/Vγ9+ lymphocytes, or an inversion of the ratio of γδ+/Vδ2+ to γδ+/Vδ2- lymphocytes or γδ+/Vγ9+ to γδ+/Vγ9- lymphocytes, relative to an immunocompromised mammal free of either of said pathogens, provides information that treatment of said immunocompromised mammal may be warranted, to control infection by at least one of said pathogens.

13. The method of claim 11 or 12 wherein ( i ) said second and third agent are detectable optically; or wherein ( ii ) said second and third agent are specific-binding proteins.

14. A method for assessing exposure of an immunocompromised mammal to an opportunistic intracellular pathogen selected from a nontuberculous mycobacterium or a sporozoan parasite, comprising the steps of:
(a) providing a tissue sample comprising lymphocytes;
(b) contacting said sample with a detectable first agent that binds selectively to lymphocytes displaying CD3, a detectable second agent that binds selectively to lymphocytes displaying CD4, and a detectable third agent that binds selectively to lymphocytes displaying CD8; and,
(c) detecting, in said sample, lymphocytes displaying a CD3+/CD4-/CD8- immunophenotype,
wherein expansion of a population of said detected lymphocytes, relative to a population thereof in an immunocompromised mammal free of said pathogen, provides information that treatment of said immunocompromised mammal may be warranted to control infection by said pathogen, said method optionally further comprising the steps of:
(d) contacting said sample with a detectable fourth agent that binds selectively to T lymphocytes displaying the αβ T-cell receptor; and,
(e) detecting, in said sample, lymphocytes displaying a CD3+/CD4-/CD8-/αβ+ immunophenotype,
said method optionally still further comprising the steps of:
(f) contacting said sample with a detectable fourth agent that binds selectively to T-lymphocytes displaying a γδ T-cell receptor; and,
(g) detecting, in said sample, lymphocytes displaying a CD3+/CD4-/CD8-/γδ+ immunophenotype,
wherein expansion of a population of said detected lymphocytes, relative to a population thereof in an immunocompromised mammal free of either of said pathogens, provides information that treatment of said immunocompromised mammal may be warranted to control infection by at least one of said pathogens.

15. The method of claim 14 further comprising the steps of:
(h) contacting said sample with a detectable fifth agent that binds selectively to T-lymphocytes displaying a γδ T cell receptor comprising a Vδ2 polypeptide or a Vγ9 polypeptide; and
(i) detecting, in said sample, lymphocytes displaying a CD3+/CD4-/CD8-/γδ+/ Vδ2- or CD3+/CD4-/CD8-/γδ+/Vγ9-immunophenotype,
wherein expansion of a population of said detected lymphocytes, relative to a population thereof in an immunocompromised mammal free of either of said pathogens, provides information that treatment of said immunocompromised mammal may be warranted to control infection by at least one of said pathogens.

16. The method of claim 14 wherein (i) said detectable agents are detectable optically; or wherein (ii) said detection is accomplished by flow cytometry.

17. Use of detectable agents that bind selectively to T-lymphocytes displaying a γδ T-cell receptor, said receptor optionally free of a polypeptide selected from Vδ2 or Vγ9, in the manufacture of a kit for assessing exposure of an immunocompromised mammal to an opportunistic pathogen selected from a nontuberculous mycobacterium or a sporozoan parasite.

18. Use according to claim 17 wherein said kit further comprises at least a second detectable agent that binds selectively to T-lymphocytes displaying CD3, CD4, CD8, or αβ T-cell receptor.

19. Use according to claim 17 or 18 wherein said kit further comprises a reference standard for assessing relative expansion of a population of detected T-lymphocytes in a tissue sample comprising lymphocytes of said immunocompromised mammal.

20. Use according to claim 17 or 18 for assessing exposure to a nontuberculous mycobacterium selected from *Mycobacterium avium complex (MAC), M. avium intracellulare, M. kansasii, M. scrofulaceum, M. ulcerans, M. marinum, M. xenopi, M. szulgai, M. simiae, M. hemophilium, M. genovensee, M. fortiutum or M. chelonei;* or for assessing exposure to a sporozoan parasite belonging to a genus selected from *Leishmania, Isospora, Toxoplasma, Plasmodium, Babesia, Cryptosporidium or Sarcocystis;* or for assessing exposure to *Toxoplasma gondii*.

21. Use according to claim 17 or 18 for ( i ) assessing exposure of a primate afflicted with an immune deficiency syndrome to said opportunistic pathogen; or ( ii ) for assessing exposure of a human afflicted with AIDS to said opportunistic pathogen, optionally wherein said human fails to exhibit symptoms normally attributable to said pathogen.

22. Use according to claim 17 wherein ( i ) said detectable agent is detectable optically; or wherein ( ii ) said detectable agent comprises biotin, avidin, an enzyme, a radionuclide, a fluorophore or a chomophore; or wherein ( iii ) said detectable agent is suitable for flow cytometry detection; or wherein ( iv ) said detectable agent comprises a fluorophore selected from fluorescence, rhodamine, phycoerythrin, phycoreythrin-sulfoindocyanine tandem conjugate (PE-Cy5), peridinin-chlorophyll or Texas red; or wherein ( v ) said detectable agent is a specific-binding protein; or wherein ( vi ) said detectable agent is an antibody.

23. A kit for immunophenotyping lymphocytes, comprising:
(a) detectable agents that bind selectively to T-lymphocytes displaying a γδ T-cell receptor, said γδ T-cell receptor optionally free of a polypeptide selected from Vδ2 or Vγ9, and
(b) a reference standard for assessing relative expansion of a population of T-lymphocytes displaying said γδ T-cell receptor, in a tissue sample comprising lymphocytes of an immunocompromised mammal suspected of being exposed to an opportunistic intracellular pathogen selected from a nontuberculous mycobacterium or a sporozoan parasite.

24. A kit for immunophenotyping lymphocytes, comprising:
(a) first, second and third detectable agents that bind to T-lymphocytes displaying, respectively, CD3, CD4 and CD8, and
(b) a reference standard for assessing relative expansion of a population of T-lymphocytes displaying a CD3+CD4-CD8- phenotype in a tissue sample comprising lymphocytes of an immunocompromised mammal suspected of being exposed to an opportunistic intracellular pathogen selected from a nontuberculous mycobacterium or a sporozoan parasite.

25. The kit of claim 23 further comprising
(c) another detectable agent that binds selectively to T-lymphocytes displaying CD8.

26. The kit of claim 24 further comprising
(c) a detectable fourth agent that binds selectively to T-lymphocytes displaying αβ T-cell receptor.

27. The kit of claim 23 or 24 wherein ( i ) each said agent is detectable optically; or wherein ( ii ) each said agent is suitable for detection by fluorescence-activated cell sorting; or wherein ( iii ) each said agent is a specific-binding protein; or wherein ( iv ) each said agent is an antibody; or wherein ( v ) each said agent further comprises biotin, avidin, an enzyme, a radionuclide, a fluorophore or a chromophore; or wherein ( vi ) each said agent further comprises a fluorophore selected from fluorescein, rhodamine, phycoerythrin, phycoerythrin-sulfoindocyanine tandem conjugate (PE-Cy5), peridinin-chlorophyll or Texas red.
